(19) Europäisches Patentamt
European Patent Office
Office européen des brevets

(11) EP 2 522 362 A1

(12) EUROPEAN PATENT APPLICATION

(43) Date of publication:
**14.11.2012 Bulletin 2012/46**

(51) Int Cl.:
***A61K 39/395*** *(2006.01)* ***C07K 16/18*** *(2006.01)*
***A61K 45/06*** *(2006.01)*

(21) Application number: **12169513.4**

(22) Date of filing: **03.02.2005**

(84) Designated Contracting States:
**AT BE BG CH CY CZ DE DK EE ES FI FR GB GR HU IE IS IT LI LT LU MC NL PL PT RO SE SI SK TR**

(30) Priority: **03.02.2004 US 771552**

(62) Document number(s) of the earlier application(s) in accordance with Art. 76 EPC:
**05712607.0 / 1 720 571**

(71) Applicant: **ALEXION PHARMACEUTICALS, INC.**
**Cheshire, CT 06410 (US)**

(72) Inventors:
- **Bell, Leonard**
  **Woodbridge, CT 06525 (US)**
- **Rother, Russel, P.**
  **Oklahoma City, OK 73151 (US)**

(74) Representative: **Vossius & Partner**
**Siebertstrasse 4**
**81675 München (DE)**

Remarks:
This application was filed on 25-05-2012 as a divisional application to the application mentioned under INID code 62.

# (54) Method of treating hemolytic disease

(57) Paroxysmal nocturnal hemoglobinuria or other hemolytic diseases are treating using a compound which binds to or otherwise blocks the generation and/or the activity of one or more complement components, such as, for example, a complement-inhibiting antibody.

EP 2 522 362 A1

## Description

### RELATED APPLICATIONS

This application claims priority to, and is a continuation-in-part of, U.S. Patent Application Serial No. 10/771,552, filed February 3, 2004, the entire disclosure of which is incorporated herein by this reference.

### BACKGROUND

#### Technical Field

This disclosure relates to a method of treating a hemolytic disease such as, for example, paroxysmal nocturnal hemoglobinuria ("PNH"), by administering a compound which binds to, or otherwise blocks, the generation and/or activity of one or more complement components.

#### Background of Related Art

Paroxysmal nocturnal hemoglobinuria ("PNH") is an uncommon blood disorder wherein red blood cells are compromised and are thus destroyed more rapidly than normal red blood cells. PNH results from a mutation of bone marrow cells resulting in the generation of abnormal blood cells. More specifically, PNH is believed to be a disorder of hematopoietic stem cells, which give rise to distinct populations of mature blood cells. The basis of the disease appears to be somatic mutations leading to the inability to synthesize the glycosyl-phosphatidylinositol ("GPI") anchor that is responsible for binding proteins to cell membranes. The mutated gene, PIG-A (phosphatidylinositol glycan class A) resides in the X chromosome and can have several different mutations, varying from deletions to point mutations.

PNH causes a sensitivity to complement proteins and this sensitivity occurs in the cell membrane. PNH cells are deficient in a number of proteins, particularly essential complement-regulating surface proteins. These complement-regulating surface proteins include the decay-accelerating factor ("DAF") or CD55 and membrane inhibitor of reactive lysis ("MIRL") or CD59.

PNH is characterized by hemolytic anemia (a decreased number of red blood cells), hemoglobinuria (the presence of hemoglobin in the urine particularly evident after sleeping), and hemoglobinemia (the presence of hemoglobin in the bloodstream). PNH-afflicted individuals are known to have paroxysms, which are defined here as incidences of dark-colored urine. Hemolytic anemia is due to intravascular destruction of red blood cells by complement components. Other known symptoms include dysphagia, fatigue, erectile dysfunction, thrombosis and recurrent abdominal pain.

Hemolysis resulting from hemolytic diseases causes local and systemic nitric oxide (NO) deficiency through the release of free hemoglobin. Free hemoglobin is a very efficient scavenger of NO, due in part to the accessibility of NO in the non-erythrocyte compartment and a $10^6$ times greater affinity of the heme moiety for NO than that for oxygen. The occurrence of intravascular hemolysis often generates sufficient free hemoglobin to completely deplete haptoglobin. Once the capacity of this hemoglobin scavenging protein is exceeded, consumption of endogenous NO ensues. For example, in a setting of intravascular hemolysis such as PNH, where LDH levels can easily exceed 2 - 3 times their normal levels, free hemoglobin would likely obtain concentrations of 0.8 -1.6 g/l. Since haptoglobin can only bind somewhere between 0.7 to 1.5 g/l of hemoglobin depending on the haptoglobin allotype, a large excess of free hemoglobin would be generated. Once the capacity of hemoglobin reabsorption by the kidney proximal tubules is exceeded, hemoglobinuria ensues. The release of free hemoglobin during intravascular hemolysis results in excessive consumption of NO with subsequent enhanced smooth muscle contraction, vasoconstriction and platelet activation and aggregation. PNH-related morbidities associated with NO scavenging by hemoglobin include abdominal pain, erectile dysfunction, esophageal spasm, and thrombosis.

The laboratory evaluation of hemolysis normally includes hematologic, serologic, and urine tests. Hematologic tests include an examination of the blood smear for morphologic abnormalities of RBCs (to determine causation), and the measurement of the reticulocyte count in whole blood (to determine bone marrow compensation for RBC loss). Serologic tests include lactate dehydrogenase (LDH; widely performed), and free hemoglobin (not widely performed) as a direct measure of hemolysis. LDH levels, in the absence of tissue damage in other organs, can be useful in the diagnosis and monitoring of patients with hemolysis. Other serologic tests include bilirubin or haptoglobin, as measures of breakdown products or scavenging reserve, respectively. Urine tests include bilirubin, hemosiderin, and free hemoglobin, and are generally used to measure gross severity of hemolysis and for differentiation of intravascular vs. extravascular etiologies of hemolysis rather than routine monitoring of hemolysis. Further, RBC numbers, RBC (i.e. cell-bound) hemoglobin, and hematocrit are generally performed to determine the extent of any accompanying anemia rather than as a measure of hemolytic activity per se.

Steroids have been employed as a therapy for hemolytic diseases and may be effective in suppressing hemolysis in some patients, although long term use of steroid therapy carries many negative side effects. Afflicted patients may require blood transfusions, which carry risks of infection. Anticoagulation therapy may also be required to prevent blood clot formation. Bone marrow transplantation has been known to cure PNH, however, bone marrow matches are often very difficult to find and mortality rates are high with such procedure.

It would be advantageous to provide a treatment which safely and reliably eliminates and/or limits hemolytic diseases, such as PNH, and their effects.

## Summary

Paroxysmal nocturnal hemoglobinuria ("PNH") and other hemolytic diseases are treated in accordance with this disclosure using a compound which binds to or otherwise blocks the generation and/or activity of one or more complement components. Suitable compounds include, for example, antibodies which bind to or otherwise block the generation and/or activity of one or more complement components, such as, for example, an antibody specific to complement component C5. In particularly useful embodiments, the compound is an anti-C5 antibody selected from the group consisting of h5G1.1-mAb (eculizumab), h5G1.1-scFv (pexelizumab) and other functional fragments of h5G1.1. It has surprisingly been found that the present methods provide improvements in the PNH subject within 24 hours of administration of the compound. For example, hemolysis is significantly reduced within 24 hours of administration of the compound as indicated by resolution of hemoglobinuria.

The complement-inhibiting compound can be administered prophylactically in individuals known to have a hemolytic disease to prevent, or help prevent the onset of symptoms. Alternatively, the complement-inhibiting compound can be administered as a therapeutic regimen to an individual experiencing symptoms of a hemolytic disease.

In another aspect, a method of increasing the proportion of complement sensitive type III red blood cells and therefore the total red blood cell count in a patient afflicted with a hemolytic disease is contemplated. The method comprises administering a compound which binds to or otherwise blocks the generation and/or activity of one or more complement components to a patient afflicted with a hemolytic disease. By increasing type III red blood cell count, symptoms such as fatigue and anemia also can be alleviated in a patient afflicted with a hemolytic disease.

In yet another aspect, the present disclosure contemplates a method of rendering a subject afflicted with a hemolytic disease less dependent on transfusions or transfusion-independent by administering a compound to the subject, the compound being selected from the group consisting of compounds which bind to one or more complement components, compounds which block the generation of one or more complement components and compounds which block the activity of one or more complement components. It has surprisingly been found that patients can be rendered transfusion-independent in accordance with the present methods. Unexpectedly, transfusion-independence can be maintained in some embodiments for twelve months or more, long beyond the 120 day life cycle of red blood cells. In other embodiments, transfusion-independence can be maintained for two years or more. Treatment for six months or more is required for the evaluation of transfusion independence given the long half life of red blood cells.

In another aspect, the present disclosure contemplates a method of treating a nitric oxide (NO) imbalance in a subject by administering a compound to the subject, the compound being selected from the group consisting of compounds which bind to one or more complement components, compounds which block the generation of one or more complement components and compounds which block the activity of one or more complement components. By reducing the lysis of red blood cells, the present methods reduce the amount of free hemoglobin in the bloodstream, thereby increasing serum levels of nitric oxide (NO). In particularly useful embodiments, NO homeostasis is restored wherein there is a resolution of symptoms attributable to NO deficiency.

In another aspect, the present disclosure contemplates a method of treating thrombosis in a subject by administering a compound to the subject, the compound being selected from the group consisting of compounds which bind to one or more complement components, compounds which block the generation of one or more complement components and compounds which block the activity of one or more complement components.

In another aspect, the present disclosure contemplates a method of treating fatigue in a subject afflicted with a hemolytic disease by administering a compound to the subject, the compound being selected from the group consisting of compounds which bind to one or more complement components, compounds which block the generation of one or more complement components and compounds which block the activity of one or more complement components.

In another aspect, the present disclosure contemplates a method of treating erectile dysfunction in a subject afflicted with a hemolytic disease by administering a compound to the subject, the compound being selected from the group consisting of compounds which bind to one or more complement components, compounds which block the generation of one or more complement components and compounds which block the activity of one or more complement components.

In another aspect, the present disclosure contemplates a method of treating abdominal pain in a subject afflicted with a hemolytic disease by administering a compound to the subject, the compound being selected from the group consisting of compounds which bind to one or more complement components, compounds which block the generation of one or more complement components and compounds which block the activity of one or more complement components.

In yet another aspect, the present disclosure contemplates a method of treating a subject afflicted with a hemolytic disease by administering: 1) one or more compounds known to increase hematopoiesis (for example, either by boosting production, eliminating stem cell destruction or eliminating stem cell inhibition) in combination with 2) a compound selected from the group consisting of compounds which bind to one or more complement components, compounds which block the generation of one or more complement components and compounds which block the activity of one or more complement components. Suitable compounds known to increase hematopoiesis include, for example, steroids, immunosuppressants (such as, cyclosporin), anti-coagulants (such as, warfarin), folic acid, iron and the like, erythropoietin (EPO) and antithymocyte globulin (ATG), antilymphocyte globulin (ALG), EPO derivatives, and darbepoetin alfa (commercially available as Aranesp® from Amgen, Inc., Thousand Oaks, CA (Aranesp® is a man-made form of EPO produced in Chinese hamster ovary (CHO) cells by recombinant DNA technology)). In particularly useful embodiments, erythropoietin (EPO) (a compound known to increase hematopoiesis), EPO derivatives, or darbepoetin alfa may be administered in combination with an anti-C5 antibody selected from the group consisting of h5G1.1-mAb, h5G1.1-scFv and other functional fragments of h5G1.1.

In yet another aspect, the present disclosure contemplates a method of treating one or more symptoms of hemolytic diseases in a subject where the proportion of type III red blood cells of the subject's total red blood cell content is greater than 10% before or during treatment, by administering a compound selected from the group consisting of compounds which bind to one or more complement components, compounds which block the generation of one or more complement components and compounds which block the activity of one or more complement components, said compound being administered alone or in combination with one or more compounds known to increase hematopoiesis, such as EPO, EPO derivatives, or darbepoetin alfa.

In yet another aspect, the present disclosure contemplates a method of treating one or more symptoms of hemolytic diseases in a subject having a platelet count above 40,000 per microliter, by administering a compound selected from the group consisting of compounds which bind to one or more complement components, compounds which block the generation of one or more complement components and compounds which block the activity of one or more complement components, said compound being administered alone or in combination with one or more compounds known to increase hematopoiesis, such as EPO, EPO derivatives, or darbepoetin alfa.

In yet another aspect, the present disclosure contemplates a method of treating one or more symptoms of a hemolytic diseases in a subject having a reticulocyte count above 80 x $10^9$ per liter, by administering a compound selected from the group consisting of compounds which bind to one or more complement components, compounds which block the generation of one or more complement components and compounds which block the activity of one or more complement components, said compound being administered alone or in combination with one or more compounds known to increase hematopoiesis, such as EPO, EPO derivatives, or darbepoetin alfa.

## Brief Description of the Drawings

Figure 1A reports biochemical parameters of hemolysis measured during treatment of PNH patients with an anti-C5 antibody.

Figure 1B graphically depicts the effect of treatment with an anti-C5 antibody on lactate dehydrogenase (LDH) levels.

Figure 2 shows a urine color scale devised to monitor the incidence of paroxysm of hemoglobinuria in PNH patients.

Figure 3 is a graph of the effects of eculizumab treatments on patient paroxysm rates, as compared to pre-treatment rates.

Figure 4 shows urine samples of PNH patients and measurements of hemoglobinuria, dysphagia, LDH, AST, pharmacokinetics (PK) and pharmacodynamics (PD) reflecting the immediate and positive effects of the present methods on hemolysis, symptoms and pharmacodynamics suitable to completely block complement.

Figure 5 graphically depicts the effect of anti-C5 antibody dosing schedule on hemoglobinuria over time.

Figures 6a and 6b are graphs comparing the number of transfusion units required per patient per month, prior to and during treatment with an anti-C5 antibody: Figure 6a depicts cytopenic patients; and Figure 6b depicts non-cytopenic patients.

Figure 7 shows the management of a thrombocytopenic patient by administering an anti-C5 antibody and erythropoietin (EPO).

Figure 8 graphically depicts the pharmacodynamics of an anti-C5 antibody.

Figure 9 is a chart of the results of European Organization for Research and Treatment of Cancer questionnaires ("EORTC QLC-C30") completed during the anti-C5 therapy regimen addressing quality of life issues.

Figure 10 is a chart depicting the effects of anti-C5 antibody treatments on adverse symptoms associated with PNH.

Detailed Description

The present disclosure relates to a method of treating paroxysmal nocturnal hemoglobinuria ("PNH") and other hemolytic diseases in mammals. Specifically, the methods of treating hemolytic diseases, which are described herein, involve using compounds which bind to or otherwise block the generation and/or activity of one or more complement components. The present methods have been found to provide surprising results. For instance, hemolysis rapidly ceases upon administration of the compound which binds to or otherwise blocks the generation and/or activity of one or more complement components, with hemoglobinuria being significantly reduced after treatment. Also, hemolytic patients can be rendered less dependent on transfusions or transfusion-independent for extended periods (twelve months or more), well beyond the 120 day life cycle of red blood cells. In addition, type III red blood cell count can be increased dramatically in the midst of other mechanisms of red blood cell lysis (non-complement mediated and/or earlier complement component mediated e.g., Cb3). Another example of a surprising result is that symptoms resolved, indicating that NO serum levels were increased enough even in the presence of other mechanisms of red blood cell lysis. These and other results reported herein are unexpected and could not be predicted from prior treatments of hemolytic diseases.

Any compound which binds to or otherwise blocks the generation and/or activity of one or more complement components can be used in the present methods. A specific class of such compounds which is particularly useful includes antibodies specific to a human complement component, especially anti-C5 antibodies. The anti-C5 antibody inhibits the complement cascade and, ultimately, prevents red blood cell ("RBC") lysis by the complement protein complex C5b-9. By inhibiting and/or reducing the lysis of RBCs, the effects of PNH and other hemolytic diseases (including symptoms such as hemoglobinuria, anemia, hemoglobinemia, dysphagia, fatigue, erectile dysfunction, recurrent abdominal pain and thrombosis) are eliminated or decreased.

In another embodiment, soluble forms of the proteins CD55 and CD59, singularly or in combination with each other, can be administered to a subject to inhibit the complement cascade in its alternative pathway. CD55 inhibits at the level of C3, thereby preventing the further progression of the cascade. CD59 inhibits the C5b-8 complex from combining with C9 to form the membrane attack complex (see discussion below).

The complement system acts in conjunction with other immunological systems of the body to defend against intrusion of bacterial and viral pathogens. There are at least 25 proteins involved in the complement cascade, which are found as a complex collection of plasma proteins and membrane cofactors. Complement components achieve their immune defensive functions by interacting in a series of intricate but precise enzymatic cleavage and membrane binding events. The resulting complement cascade leads to the production of products with opsonic, immunoregulatory, and lytic functions. A concise summary of the biologic activities associated with complement activation is provided, for example, in The Merck Manual, $16^{th}$ Edition.

The complement cascade progresses via the classical pathway, the alternative pathway or the lectin pathway. These pathways share many components, and while they differ in their initial steps, they converge and share the same "terminal complement" components (C5 through C9) responsible for the activation and destruction of target cells. The classical complement pathway is typically initiated by antibody recognition of and binding to an antigenic site on a target cell. The alternative pathway is usually antibody independent, and can be initiated by certain molecules on pathogen surfaces. Additionally, the lectin pathway is typically initiated with binding of mannose-binding lectin ("MBL") to high mannose substrates. These pathways converge at the point where complement component C3 is cleaved by an active protease to yield C3a and C3b.

C3a is an anaphylatoxin (see discussion below). C3b binds to bacteria and other cells, as well as to certain viruses and immune complexes, and tags them for removal from the circulation. (C3b in this role is known as opsonin.) The opsonic function of C3b is generally considered to be the most important anti-infective action of the complement system. Patients with genetic lesions that block C3b function are prone to infection by a broad variety of pathogenic organisms, while patients with lesions later in the complement cascade sequence, i.e., patients with lesions that block C5 functions, are found to be more prone only to Neisseria infection, and then only somewhat more prone (Fearon, in Intensive Review of Internal Medicine, $2^{nd}$ Ed. Fanta and Minaker, eds. Brigham and Women's and Beth Israel Hospitals, 1983).

C3b also forms a complex with other components unique to each pathway to form classical or alternative C5 convertase, which cleaves C5 into C5a and C5b. C3 is thus regarded as the central protein in the complement reaction sequence since it is essential to all three activation pathways (Wurzner, et al., Complement Inflamm. 8:328-340, 1991). This property of C3b is regulated by the serum protease Factor I, which acts on C3b to produce iC3b (inactive C3b). While still functional as an opsonin, iC3b can not form an active C5 convertase.

The pro-C5 precursor is cleaved after amino acid 655 and 659, to yield the beta chain as an amino terminal fragment (amino acid residues +1 to 655 of the sequence) and the alpha chain as a carboxyl terminal fragment (amino acid residues 660 to 1658 of the sequence), with four amino acids (amino acid residues 656-659 of the sequence) deleted between the two. C5 is glycosylated, with about 1.5-3 percent of its mass attributed to carbohydrate. Mature C5 is a heterodimer of a 999 amino acid 115 kDa alpha chain that is disulfide linked to a 655 amino acid 75 kDa beta chain.

C5 is found in normal serum at approximately 75 μg/ml (0.4 μM). C5 is synthesized as a single chain precursor protein product of a single copy gene (Haviland et al. J. Immunol. 1991, 146:362-368). The cDNA sequence of the transcript of this gene predicts a secreted pro-C5 precursor of 1658 amino acids along with an 18 amino acid leader sequence (see, U.S. patent 6,355,245).

Cleavage of C5 releases C5a, a potent anaphylatoxin and chemotactic factor, and leads to the formation of the lytic terminal complement complex, C5b-9. C5a is cleaved from the alpha chain of C5 by either alternative or classical C5 convertase as an amino terminal fragment comprising the first 74 amino acids of the alpha chain (i.e., amino acid residues 660-733 of the sequence). Approximately 20 percent of the 11 kDa mass of C5a is attributed to carbohydrate. The cleavage site for convertase action is at, or immediately adjacent to, amino acid residue 733 of the sequence. A compound that binds at, or adjacent, to this cleavage site would have the potential to block access of the C5 convertase enzymes to the cleavage site and thereby act as a complement inhibitor.

C5b combines with C6, C7, and C8 to form the C5b-8 complex at the surface of the target cell. Upon binding of several C9 molecules, the membrane attack complex ("MAC", C5b-9, terminal complement complex-TCC) is formed. When sufficient numbers of MACs insert into target cell membranes, the openings they create (MAC pores) mediate rapid osmotic lysis of the target cells. Lower, non-lytic concentrations of MACs can produce other proinflammatory effects. In particular, membrane insertion of small numbers of the C5b-9 complexes into endothelial cells and platelets can cause deleterious cell activation. In some cases activation may precede cell lysis.

C5a and C5b-9 also have pleiotropic cell activating properties, by amplifying the release of downstream inflammatory factors, such as hydrolytic enzymes, reactive oxygen species, arachidonic acid metabolites and various cytokines. C5 can also be activated by means other than C5 convertase activity. Limited trypsin digestion (Minta and Man, J. Immunol. 1977, 119:1597-1602; Wetsel and Kolb, J. Immunol. 1982, 128:2209-2216) and acid treatment (Yammamoto and Gewurz, J. Immunol. 1978, 120:2008; Damerau et al., Molec. Immunol. 1989, 26:1133-1142) can also cleave C5 and produce active C5b.

As mentioned above, C3a and C5a are anaphylatoxins. These activated complement components can trigger mast cell degranulation, which releases histamine and other mediators of inflammation, resulting in smooth muscle contraction, increased vascular permeability, leukocyte activation, and other inflammatory phenomena including cellular proliferation resulting in hypercellularity. C5a also functions as a chemotactic peptide that serves to attract pro-inflammatory granulocytes to the site of complement activation.

Any compounds which bind to or otherwise block the generation and/or activity of any of the human complement components may be utilized in accordance with the present disclosure. In some embodiments, antibodies specific to a human complement component are useful herein. Some compounds include antibodies directed against complement components C-1, C-2, C-3, C-4, C-5, C-6, C-7, C-8, C-9, Factor D, Factor B, Factor P, MBL, MASP-1, and MASP-2, thus preventing the generation of the anaphylatoxic activity associated with C5a and/or preventing the assembly of the membrane attack complex associated with C5b.

Also useful in the present methods are naturally occurring or soluble forms of complement inhibitory compounds such as CR1, LEX-CR1, MCP, DAF, CD59, Factor H, cobra venom factor, FUT-175, complestatin, and K76 COOH. Other compounds which may be utilized to bind to or otherwise block the generation and/or activity of any of the human complement components include, but are not limited to, proteins, protein fragments, peptides, small molecules, RNA aptamers including ARC187 (which is commercially available from Archemix Corp., Cambridge, MA), L-RNA aptamers, spiegelmers, antisense compounds, serine protease inhibitors, molecules which may be utilized in RNA interference (RNAi) such as double stranded RNA including small interfering RNA (siRNA), locked nucleic acid (LNA) inhibitors, peptide nucleic acid (PNA) inhibitors, etc.

Functionally, one suitable class of compounds inhibits the cleavage of C5, which blocks the generation of potent proinflammatory molecules C5a and C5b-9 (terminal complement complex). Preferably, the compound does not prevent the formation of C3b, which subserves critical immunoprotective functions of opsonization and immune complex clearance.

While preventing the generation of these membrane attack complex molecules, inhibition of the complement cascade at C5 preserves the ability to generate C3b, which is critical for opsonization of many pathogenic microorganisms, as well as for immune complex solubilization and clearance. Retaining the capacity to generate C3b appears to be particularly important as a therapeutic factor in complement inhibition for hemolytic diseases, where increased susceptibility to thrombosis, infection, fatigue, lethargy and impaired clearance of immune complexes are pre-existing clinical features of the disease process.

Particularly useful compounds for use herein are antibodies that reduce, directly or indirectly, the conversion of complement component C5 into complement components C5a and C5b. One class of useful antibodies are those having at least one antigen binding site and exhibiting specific binding to human complement component C5. Particularly useful complement inhibitors are compounds which reduce the generation of C5a and/or C5b-9 by greater than about 30%. Anti-C5 antibodies that have the desirable ability to block the generation of C5a have been known in the art since at least 1982 (Moongkarndi et al. Immunobiol. 1982, 162:397; Moongkarndi et al. Immunobiol. 1983, 165:323). Antibodies known in the art that are immunoreactive against C5 or C5 fragments include antibodies against the C5 beta chain (Moongkarndi et al. Immunobiol. 1982, 162:397; Moongkarndi et al. Immunobiol. 1983, 165:323; Wurzner et al. 1991, supra; Mollnes et al. Scand. J. Immunol. 1988, 28:307-312); C5a (see for example, Ames et al. J. Immunol. 1994, 152: 4572-4581, U.S. Pat. No. 4,686,100, and European patent publication No. 0 411 306); and antibodies against non-human C5 (see for example, Giclas et al. J. Immunol. Meth. 1987, 105:201-209). Particularly useful anti-C5 antibodies are h5G1.1-mAb, h5G1.1-scFv and other functional fragments of h5G1.1. Methods for the preparation of h5G1.1-mAb, h5G1.1-scFv and other functional fragments of h5G1.1 are described in U.S. Patent No. 6,355,245 and "inhibition of Complement Activity by Humanized Anti-C5 Antibody and Single Chain Fv", Thomas et al., Molecular Immunology, Vol. 33, No. 17/18, pages 1389-1401, 1996, the disclosures of which are incorporated herein in their entirety by this reference. The antibody h5G1.1-mAb is currently undergoing clinical trials under the tradename eculizumab.

Hybridomas producing monoclonal antibodies reactive with complement component C5 can be obtained according to the teachings of Sims, et al., U.S. Pat. No. 5,135,916. Antibodies are prepared using purified components of the complement C5 component as immunogens according to known methods. In accordance with this disclosure, complement component C5, C5a or C5b is preferably used as the immunogen. In accordance with particularly preferred useful embodiments, the immunogen is the alpha chain of C5.

Particularly useful antibodies share the required functional properties discussed in the preceding paragraph and have any of the following characteristics:

(1) they compete for binding to portions of C5 that are specifically immunoreactive with 5G1.1;
(2) they specifically bind to the C5 alpha chain -- such specific binding, and competition for binding can be determined by various methods well known in the art, including the plasmon surface resonance method (Johne et al., J. Immunol. Meth. 1993, 160:191-198); and
(3) they block the binding of C5 to either C3 or C4 (which are components of the C5 convertases).

The compound that inhibits the production and/or activity of at least one complement component can be administered in a variety of unit dosage forms. The dose will vary according to the particular compound employed. For example, different antibodies may have different masses and/or affinities, and thus require different dosage levels. Antibodies prepared as fragments (e.g., Fab, $F(ab')_2$, scFv) will also require differing dosages than the equivalent intact immunoglobulins, as they are of considerably smaller mass than intact immunoglobulins, and thus require lower dosages to reach the same molar levels in the patient's blood.

The dose will also vary depending on the manner of administration, the particular symptoms of the patient being treated, the overall health, condition, size, and age of the patient, and the judgment of the prescribing physician.

Administration of the compound that inhibits the production and/or activity of at least one complement component will generally be in an aerosol form with a suitable pharmaceutical carrier, via intravenous infusion by injection, subcutaneous injection, orally, or sublingually. Other routes of administration may be used if desired.

It is further contemplated that a combination therapy can be used wherein a complement-inhibiting compound is administered in combination with a regimen of known therapy for hemolytic disease. Such regimens include administration of 1) one or more compounds known to increase hematopoiesis (for example, either by boosting production, eliminating stem cell destruction or eliminating stem cell inhibition) in combination with 2) a compound selected from the group consisting of compounds which bind to one or more complement components, compounds which block the generation of one or more complement components and compounds which block the activity of one or more complement components. Suitable compounds known to increase hematopoiesis include, for example, steroids, immunosuppressants (such as, cyclosporin), anti-coagulants (such as, warfarin), folic acid, iron and the like, erythropoietin (EPO) and antithymocyte globulin (ATG), antilymphocyte globulin (ALG), EPO derivatives, and darbepoetin alfa (commercially available as Aranesp® from Amgen, Inc., Thousand Oaks, CA (Aranesp® is a man-made form of EPO produced in Chinese hamster ovary (CHO) cells by recombinant DNA technology)). In particularly useful embodiments, erythropoietin (EPO) (a compound known to increase hematopoiesis), EPO derivatives, or darbepoetin alfa may be administered in combination with an anti-C5 antibody selected from the group consisting of h5G1.1-mAb, h5G1.1-scFv and other functional fragments of h5G1.1.

Formulations suitable for injection are found in Remington's Pharmaceutical Sciences, Mack Publishing Company, Philadelphia, Pa., 17th ed. (1985). Such formulations must be sterile and non-pyrogenic, and generally will include a pharmaceutically effective carrier, such as saline, buffered (e.g., phosphate buffered) saline, Hank's solution, Ringer's solution, dextrose/saline, glucose solutions, and the like. The formulations may contain pharmaceutically acceptable auxiliary substances as required, such as, tonicity adjusting agents, wetting agents, bactericidal agents, preservatives, stabilizers, and the like.

The present disclosure contemplates methods of reducing hemolysis in a patient afflicted with a hemolytic disease by administering one or more compounds which bind to or otherwise block the generation and/or activity of one or more complement components. Reducing hemolysis means that the duration of time a person suffers from hemolysis is reduced by about 25% or more. The effectiveness of the treatment can be evaluated in any of the various manners known to those skilled in the art for determining the level of hemolysis in a patient. One qualitative method for detecting hemolysis is to observe the occurrences of hemoglobinuria. Quite surprisingly, treatment in accordance with the present methods reduces hemolysis as determined by a rapid reduction in hemoglobinuria.

A more qualitative manner of measuring hemolysis is to measure lactate dehydrogenase (LDH) levels in the patient's bloodstream. LDH catalyzes the interconversion of pyruvate and lactate. Red blood cells metabolize glucose to lactate, which is released into the blood and is taken up by the liver. LDH levels are used as an objective indicator of hemolysis. As those skilled in the art will appreciate, measurements of "upper limit of normal" levels of LDH will vary from lab to lab depending on a number of factors including the particular assay employed and the precise manner in which the assay is conducted. Generally speaking, however, the present methods can reduce hemolysis in a patient afflicted with a hemolytic disease as reflected by a reduction of LDH levels in the patients to within 20% of the upper limit of normal LDH levels. Alternatively, the present methods can reduce hemolysis in a patient afflicted with a hemolytic disease as reflected by a reduction of LDH levels in the patients of greater than 50% of the patient's pre-treatment LDH level, preferably greater than 65% of the patient's pre-treatment LDH level, most preferably greater than 80% of the patient's pre-treatment LDH level.

Another quantitative measurement of a reduction in hemolysis is the presence of GPI-deficient red blood cells (Type III red blood cells). As those skilled in the art will appreciate, Type III red blood cells have no GPI-anchor protein expression on the cell surface. The proportion of GPI-deficient cells can be determined by flow cytometry using, for example, the technique described in Richards, et al., Clin. Appl. Immunol. Rev., vol. 1, pages 315-330, 2001. The present methods can reduce hemolysis in a patient afflicted with a hemolytic disease as reflected by an increase in Type III red blood cells. Preferably an increase in Type III red blood cell levels in the patient of greater than 25% of the total red blood cell count is achieved, more preferably an increase in Type III red blood cell levels in the patients greater than 50% of the total red blood cell count is achieved, most preferably an increase in Type III red blood cell levels in the patients greater than 75% of the total red blood cell count is achieved.

Methods of reducing one or more symptoms associated with PNH or other hemolytic diseases are also within the scope of the present disclosure. Such symptoms include, for example, abdominal pain, fatigue, dyspnea and insomnia. Symptoms can be the direct result of lysis of red blood cells (e.g., hemoglobinuria, anemia, fatigue, low red blood cell count, etc.) or the symptoms can result from low nitric oxide (NO) levels in the patient's bloodstream (e.g., abdominal pain, erectile dysfunction, dysphagia, thrombosis, etc.). It has recently been reported that almost all patients with greater than 40% PNH type III granulocyte clone have thrombosis, abdominal pain, erectile dysfunction and dysphagia, indicating a high hemolytic rate (see Moyo et al., British J. Haematol. 26:133-138 (2004)).

In particularly useful embodiments, the present methods provide a reduction in one or more symptoms associated with PNH or other hemolytic diseases in a patient having a platelet count in excess of 40,000 per microliter (a hypoplastic patient), preferably in excess of 75,000 per microliter, most preferably in excess of 150,000 per microliter. In other embodiments, the present methods provide a reduction in one or more symptoms associated with PNH or other hemolytic diseases in a patient where the proportion of PNH type III red blood cells of the subject's total red blood cell content is greater than 10%, preferably greater than 25%, most preferably in excess of 50%. In yet other embodiments, the present methods provide a reduction in one or more symptoms associated with PNH or other hemolytic diseases in a patient having a reticulocyte count in excess of 80 x $10^9$ per liter, more preferably in excess of 120 x $10^9$ per liter, most preferably in excess of 150 x $10^9$ per liter. Patients in the most preferable ranges recited above have active bone marrow and will produce adequate numbers of red blood cells. While in a patient afflicted with PNH or other hemolytic disease the red blood cells may be defective in one or more ways (e.g., GPI deficient), the present methods are particularly useful in protecting such cells from lysis resulting from complement activation. Thus, patients within the preferred ranges benefit most from the present methods.

In one aspect, a method of reducing fatigue is contemplated, the method including the step of administering to a subject having or susceptible to a hemolytic disease a compound which binds to or otherwise blocks the generation and/or activity of one or more complement components. Reducing fatigue means the duration of time a person suffers from fatigue is reduced by about 25% or more. Fatigue is a symptom believed to be associated with intravascular hemolysis as the fatigue relents when hemoglobinuria resolves even in the presence of anemia. By reducing the lysis of red blood cells, the present methods reduce fatigue. Patients within the above-mentioned preferred ranges of type III red blood cells, reticulocytes and platelets benefit most from the present methods.

In another aspect, a method of reducing abdominal pain is contemplated, the method including the step of administering to a subject having or susceptible to a hemolytic disease a compound which binds to or otherwise blocks the generation and/or activity of one or more complement components. Reducing abdominal pain means the duration of time a person suffers from abdominal pain is reduced by about 25% or more. Abdominal pain is a symptom resulting from the inability of a patient's natural levels of haptoglobin to process all the free hemoglobin released into the bloodstream as a result of intravascular hemolysis, resulting in the scavenging of NO and intestinal dystonia and spasms. By reducing the lysis of red blood cells, the present methods reduce the amount of free hemoglobin in the bloodstream, thereby reducing abdominal pain. Patients within the above-mentioned preferred ranges of type III red blood cells, reticulocytes and platelets benefit most from the present methods.

In another aspect, a method of reducing dysphagia is contemplated, the method including the step of administering to a subject having or susceptible to a hemolytic disease a compound which binds to or otherwise blocks the generation and/or activity of one or more complement components. Reducing dysphagia means the duration of time a person has dysphagia attacks is reduced by about 25% or more. Dysphagia is a symptom resulting from the inability of a patient's natural levels of haptoglobin to process all the free hemoglobin released into the bloodstream as a result of intravascular hemolysis, resulting in the scavenging of NO and esophageal spasms. By reducing the lysis of red blood cells, the present methods reduce the amount of free hemoglobin in the bloodstream, thereby reducing dysphagia. Patients within the above-mentioned preferred ranges of type III red blood cells, reticulocytes and platelets benefit most from the present methods.

in yet another aspect, a method of reducing erectile dysfunction is contemplated, the method including the step of administering to a subject having or susceptible to a hemolytic disease a compound which binds to or otherwise blocks the generation and/or activity of one or more complement components. Reducing erectile dysfunction means the duration of time a person suffers from erectile dysfunction is reduced by about 25% or more. Erectile dysfunction is a symptom believed to be associated with scavenging of NO by free hemoglobin released into the bloodstream as a result of intravascular hemolysis. By reducing the lysis of red blood cells, the present methods reduce the amount of free hemoglobin in the bloodstream, thereby increasing serum levels of NO and reducing erectile dysfunction. Patients within the above-mentioned preferred ranges of type III red blood cells, reticulocytes and platelets benefit most from the present methods.

In yet another aspect, a method of reducing hemoglobinuria is contemplated, the method including the step of administering to a subject having or susceptible to a hemolytic disease a compound which binds to or otherwise blocks the generation and/or activity of one or more complement components. Reducing hemoglobinuria means a reduction in the number of times a person has red, brown, or darker urine, wherein the reduction is typically about 25% or more. Hemoglobinuria is a symptom resulting from the inability of a patient's natural levels of haptoglobin to process all the free hemoglobin released into the bloodstream as a result of intravascular hemolysis. By reducing the lysis of red blood cells, the present methods reduce the amount of free hemoglobin in the bloodstream and urine thereby reducing hemoglobinuria. Quite surprisingly, the reduction in hemoglobinuria occurs rapidly. Patients within the above-mentioned preferred ranges of type III red blood cells, reticulocytes and platelets benefit most from the present methods.

In still another aspect, a method of reducing thrombosis is contemplated, the method including the step of administering to a subject having or susceptible to a hemolytic disease a compound which binds to or otherwise blocks the generation and/or activity of one or more complement components. Reducing thrombosis means the duration of time a person has thrombosis attacks is reduced by about 25% or more. Thrombosis is a symptom believed to be associated with scavenging of NO by free hemoglobin released into the bloodstream as a result of intravascular hemolysis and/or the lack of CD59 on the surface of platelets resulting in terminal complement mediated activation of the platelet. By reducing the lysis of red blood cells, the present methods reduce the amount of free hemoglobin in the bloodstream, thereby increasing serum levels of NO and reducing thrombosis. In addition, blockade of complement will prevent terminal complement-mediated activation of platelets and thrombosis. C5a will also be inhibited by this method which can induce platelet aggregation through C5a receptors on platelets and endothelial cells.

Thrombosis is thought to be multi-factorial in etiology including NO scavenging by free hemoglobin, the absence of terminal complement inhibition on the surface of circulating platelets and changes in the endothelium surface by cell free heme. The intravascular release of free hemoglobin may directly contribute to small vessel thrombosis. NO has been shown to inhibit platelet aggregation, induce disaggregation of aggregated platelets and inhibit platelet adhesion. Conversely, NO scavenging by hemoglobin or the reduction of NO generation by the inhibition of arginine metabolism results in an increase in platelet aggregation. PNH platelets also lack the terminal complement inhibitor CD59 and multiple studies have shown that deposition of terminal complement (C5b-9) on platelets causes membrane vesiculation and the generation of microvesicles. The microvesicles act as a site for the generation of the clotting components factor Va, Xa or the prothrombinase complex. It is thought that these particles may also contribute to the genesis of thrombosis in PNH. By reducing the lysis of red blood cells, the present methods reduce the amount of free hemoglobin in the bloodstream, thereby increasing serum levels of NO and reducing thrombosis. In addition, inhibiting complement at C5 will prevent C5b-9 and C5a mediated activation of platelets and/or endothelial cells.

In particularly useful embodiments, the present methods reduce thrombosis, especially patients having a platelet count in excess of 40,000 per microliter, preferably in excess of 75,000 per microliter, most preferably in excess of 150,000 per microliter. In other embodiments, the present methods reduce thrombosis in patients where the proportion of PNH type III red blood cells of the subject's total red blood cell content is greater than 10%, preferably greater than 25%, more preferably in excess of 50%, most preferably in excess of 75%. In yet other embodiments, the present methods reduce transfusion thrombosis in patients having a reticulocyte count in excess of $80 \times 10^9$ per liter, more preferably in excess of $120 \times 10^9$ per liter, most preferably in excess of $150 \times 10^9$ per liter.

In still another aspect, a method of reducing anemia is contemplated, the method including the step of administering to a subject having or susceptible to a hemolytic disease a compound which binds to or otherwise blocks the generation and/or activity of one or more complement components. Reducing anemia means the duration of time a person has anemia is reduced by about 25% or more. Anemia in hemolytic diseases results from the blood's reduced capacity to carry oxygen due to the loss of red blood cell mass. By reducing the lysis of red blood cells, the present methods assist red blood cell levels to increase thereby reducing anemia.

In another aspect, a method of increasing the proportion of complement sensitive type III red blood cells and therefore the total red blood cell count in a patient afflicted with a hemolytic disease is contemplated. By increasing the patient's RBC count, fatigue, anemia and the patient's need for blood transfusions is reduced. The reduction in transfusions can be in frequency of transfusions, amount of blood units transfused, or both.

The method of increasing red blood cell count in a patient afflicted with a hemolytic disease includes the step of administering a compound which binds to or otherwise blocks the generation and/or activity of one or more complement components to a patient afflicted with a hemolytic disease. In particularly useful embodiments, the present methods increase red blood cell count in a patient afflicted with a hemolytic disease, especially patients having a platelet count in excess of 40,000 per microliter, preferably in excess of 75,000 per microliter, most preferably in excess of 150,000 per microliter. In other embodiments, the present methods increase red blood cell count in a patient afflicted with a hemolytic disease where the proportion of PNH type III red blood cells of the subject's total red blood cell content is greater than 10%, preferably greater than 25%, more preferably in excess of 50%, most preferably in excess of 75%. In yet other embodiments, the present methods increase red blood cell count in a patient afflicted with a hemolytic disease having a reticulocyte count in excess of 80 x $10^9$ per liter, more preferably in excess of 120 x $10^9$ per liter, most preferably in excess of 150 x $10^9$ per liter. in some embodiments, the methods of the present disclosure may result in a decrease in the frequency of transfusions by about 50%, typically a decrease in the frequency of transfusions by about 70%, more typically a decrease in the frequency of transfusions by about 90%.

In yet another aspect, the present disclosure contemplates a method of rendering a subject afflicted with a hemolytic disease less dependent on transfusions or transfusion-independent by administering a compound to the subject, the compound being selected from the group consisting of compounds which bind to one or more complement components, compounds which block the generation of one or more complement components and compounds which block the activity of one or more complement components. As those skilled in the art will appreciate, the normal life cycle for a red blood cell is about 120 days. Treatment for six months or more is required for the evaluation of transfusion independence given the long half life of red blood cells. It has unexpectedly been found that in some patients transfusion-independence can be maintained for twelve months or more, in some cases more than two years, long beyond the 120 day life cycle of red blood cells. In particularly useful embodiments, the present methods provide decreased dependence on transfusions or transfusion-independence in a patient afflicted with a hemolytic disease, especially patients having a platelet count in excess of 40,000 per microliter, preferably in excess of 75,000 per microliter, most preferably in excess of 150,000 per microliter. In other embodiments, the present methods provide decreased dependence on transfusions or transfusion-independence in a patient afflicted with a hemolytic disease where the proportion of PNH type III red blood cells of the subject's total red blood cell content is greater than 10%, preferably greater than 25%, more preferably in excess of 50%, most preferably in excess of 75%. In yet other embodiments, the present methods provide decreased dependence on transfusions or transfusion-independence in a patient afflicted with a hemolytic disease having a reticulocyte count in excess of 80 x $10^9$ per liter, more preferably in excess of 120 x $10^9$ per liter, most preferably in excess of 150 x $10^9$ per liter.

Methods of increasing the nitric oxide (NO) levels in a patient having PNH or some other hemolytic disease are also within the scope of the present disclosure. These methods of increasing NO levels include the step of administering to a subject having or susceptible to a hemolytic disease a compound which binds to or otherwise blocks the generation and/or activity of one or more complement components. Low NO levels arise in patients afflicted with PNH or other hemolytic diseases as a result of scavenging of NO by free hemoglobin released into the bloodstream as a result of intravascular hemolysis. By reducing the lysis of red blood cells, the present methods reduce the amount of free hemoglobin in the bloodstream, thereby increasing serum levels of NO. In particularly useful embodiments, NO homeostasis is restored as evidenced by a resolution of symptoms attributable to NO deficiencies.

## EXAMPLES

Eleven patients participated in therapy trials to evaluate the effects of anti-C5 antibody on PNH and symptoms associated therewith. PNH patients were transfusion-dependent and hemolytic. Patients were defined as transfusion dependent with a history of four or more transfusions within twelve months. The median number of transfusions within the patient pool was nine in the previous twelve months. The median number of transfusion units used in the previous twelve months was twenty-two for the patient pool.

Over the course of four weeks, each of 11 patients received a weekly 600 mg intravenous infusion of anti-C5 antibody for approximately thirty minutes. The specific anti-C5 antibody used in the study was eculizumab. Patients received 900 mg of eculizumab 1 week later then 900 mg on a biweekly basis. The first twelve weeks of the study constituted the pilot study. Following completion of the initial acute phase twelve week study, all patients participated in an extension study conducted to a total of 64 weeks. Ten of the eleven patients participated in an extension study conducted to a total of two years.

The effect of anti-C5 antibody treatments on PNH type III red blood cells ("RBCs") was tested. "PNH Type" refers to the density of GPI-anchored proteins expressed on the cell surface. Type I is normal expression, Type II is intermediate expression, and Type III has no GPI-anchor protein expression on the cell surface. The proportion of GPI-deficient cells is determined by flow cytometry in the manner described in Richards, et al., Clin. Appl. Immunol. Rev., vol. 1, pages 315-330, 2001. As compared to pre-therapy conditions, PNH Type III red blood cells increased more than 50% during the extension study. The increase from a pre-study mean value of 36.7% of all red blood cells to a 64 week mean value of 58.4% of Type III red blood cells indicated that hemolysis had decreased sharply. See Table 1, below. Eculizumab therapy protected PNH type III RBCs from complement-mediated lysis, prolonging the cells survival. This protection of the PNH-affected cells reduced the need for transfusions, paroxysms and overall hemolysis in all patients in the trial.

Table 1

| PNH Cell Populations Pre- and Post- Eculizumab Treatment in All Patients | | | | |
|---|---|---|---|---|
| | Proportion of PNH Cells (%) | | | |
| PNH Cell Type | baseline | 12 weeks | 64 weeks | p-value[a] |
| Type III RBCs | 36.7 +/- 5.9 | 59.2 +/- 8,0 | 58.4 +/- 8.5 | 0.005 |
| Type II RBCs | 5.3+/-1.4 | 7.5+/-2.1 | 13.2+/-2.4 | 0.013 |
| Type III WBCs | 92.1 +/- 4.6 | 89.9 +/- 6.6 | 91.1 +/-5.8 | N.S. |
| Type III Platelets | 92.4 +/- 2.4 | 93.3 +/- 2.8 | 92.8+/-2.6 | N.S. |
| [a]comparison of mean change from baseline to 64 weeks | | | | |

During the course of the two year extension study, it was found that PNH red cells with a complete deficiency of GPI-linked proteins (Type III red cells) progressively increased during the treatment period from a mean of 36.7% to 58.9% (p=0.001) while partially deficient PNH red cells (Type II) increased from 5.3% to 8.7% (p=0.01). There was no concomitant change in the proportion of PNH neutrophils in any of the patients during eculizumab therapy, indicating that the increase in the proportion of PNH red cells was due to a reduction in hemolysis and transfusions rather than a change in the PNH clone(s) themselves.

The effect of anti-C5 antibody treatments on lactate dehydrogenase levels ("LDH") was measured on all eleven patients. LDH catalyzes the interconversion of pyruvate and lactate. Red blood cells metabolize glucose to lactate, which is released into the blood and is taken up by the liver. LDH levels are used as an objective indicator of hemolysis. The LDH levels were decreased by greater than 80% as compared to pre-treatment levels. The LDH levels were lowered from a pre-study mean value of 3111 U/L to a mean value of 594 U/L during the pilot study and a mean value of 622 U/L after 64 weeks (p = 0.002 for 64 week comparison; see Figures 1A and 1B).

Similarly, aspartate aminotransferase (AST) levels, another marker of red blood cell hemolysis, decreased from a mean baseline value of 76 IU/L to 26 IU/L and 30 IU/L during the 12 and 64 weeks of treatment, respectively (p=0.02 for 64 week comparison). Levels of haptoglobin, hemoglobin and bilirubin, and numbers of reticulocytes, did not change significantly from prestudy values during the 64 week treatment period.

Paroxysm rates were measured and compared to pre-treatment levels. Paroxysm as used in this disclosure is defined as incidences of dark-colored urine with a colorimetric level of 6 of more on a scale of 1-10. Figure 2 shows the urine color scale devised to monitor the incidence of paroxysm of hemoglobinuria in patients with PNH before and during treatment. As compared to pre-treatment levels, the paroxysm percentage rate was reduced by 93% (see, Figure 3) from 3.0 paroxysms per patient per month before eculizumab treatment to 0.1 paroxysms per patient per month during the initial 12 weeks and 0.2 paroxysms per patient per month during the 64 week treatment (Figure 3 (p< 0.001)).

Serum hemolytic activity in nine of the eleven patients was completely blocked throughout the 64 week treatment period with trough levels of eculizumab at equilibrium ranging from approximately 35 μg/ml to 350 μg/ml. During the extension study, 2 patients did not sustain levels of eculizumab necessary to consistently block complement. This breakthrough in serum hemolytic activity occurred in the last 2 days of the 14 day dosing interval, a pattern that was repeated between multiple doses. In one of the patients, as seen in Figure 4, break-through of complement blockade resulted in hemoglobinuria, dysphagia, and increased LDH and AST, which correlated with the return of serum hemolytic activity. At the next dose, symptoms resolved (Figure 5) and reduction in the dosing interval from 900 mg every 14 days to 900 mg every 12 days resulted in a regain of complement control which was maintained over the extension study to 64 weeks in both patients. This patient showed a 24 hour resolution of dysphagia and hemoglobinuria. A reduction in the dosing interval from 14 to 12 days was sufficient to maintain levels of eculizumab above 35 μg/ml and effectively and consistently blocked serum hemolytic activity for the remainder of the extension study for both patients.

The patients' need for transfusions was also reduced by the treatment with eculizumab. Figure 6a compares the number of transfusion units required per patient per month, prior to and during treatment with an anti-C5 antibody for cytopenic patients, while Figure 6b compares the number of transfusion units required per patient per month, prior to and during treatment with an anti-C5 antibody for non-cytopenic patients. A significant reduction in the need for transfusion was also noted in the entire group (mean transfusion rates decreased from 2.1 units per patient per month during a 1 year period prior to treatment to 0.6 units per patient per month during the initial 12 weeks and 0.5 units per patient per month during the combined 64 week treatment period), with non-cytopenic patients benefiting the most. In fact, four of the non-thrombocytopenic patients with normal platelet counts (≥150,000 per microliter) became transfusion-independent during the 64 week treatment.

The effect of eculizumab administered in combination with erythropoietin (EPO) was also evaluated in a thrombocytopenic patient. EPO (NeoRecormon®, Roche Pharmaceuticals, Basel, Switzerland) was administered in an amount of 18,000 I.U. three times per week beginning in week 23 of the study. As shown in Figure 7, the frequency of transfusions required for this patient was significantly reduced, and soon halted.

For the two year extension study, 10 of the 11 patients from the initial 3 month study continued to receive 900 mg of eculizumab every other week. (One patient discontinued eculizumab therapy after 23 months.) Six of the 11 patients had normal platelets (no clinical evidence of marrow failure) whereas 5 of the 11 had low platelets. For the patient who discontinued eculizumab therapy after 23 months, intravascular hemolysis was successfully controlled by eculizumab, but the patient continued to be transfused even after erythropoietin therapy. This patient had the most severe hypoplasia at the start of eculizumab therapy with a platelet count below $30 \times 10^9$/l, suggesting that the ongoing transfusions were likely a result of the underlying bone marrow failure.

Results of the two year extension study also demonstrated that there was a statistically significant decrease in transfusion requirements for the patients. Three patients remained transfusion independent during the entire two year treatment period, and four cytopenic patients became transfusion independent, three following treatment with EPO (NeoRecormon®). The reduction in transfusion requirements was found to be most pronounced in patients with a good marrow reserve.

Pharmacodynamic levels were measured and recorded according to eculizumab doses. The pharmacodynamic analysis of eculizumab was determined by measuring the capacity of patient serum samples to lyse chicken erythrocytes in a standard total human serum complement hemolytic assay. Briefly, patient samples or human control serum (Quidel, San Diego CA) was diluted to 40% vol/vol with gelatin veronal-buffered saline (GVB2+, Advanced Research technologies, San Diego, CA) and added in triplicate to a 96-well plate such that the final concentrations of serum in each well was 20%. The plate was then incubated at room temperature while chicken erythrocytes (Lampire Biologics, Malvern, PA) were washed. The chicken erythrocytes were sensitized by the addition of anti-chicken red blood cell polyclonal antibody (0.1 % vol/vol). The cells were then washed and resuspended in GVB2+ buffer. Chicken erythrocytes ($2.5 \times 10^6$ cells/30 μL) were added to the plate containing human control serum or patient samples and incubated at 37°C for 30 min. Each plate contained six additional wells of identically prepared chicken erythrocytes of which four wells were incubated with 20% serum containing 2 mM EDTA as the blank and two wells were incubated with GVB2+ buffer alone as a negative control for spontaneous hemolysis. The plate was then centrifuged and the supernatant transferred to a new flat bottom 96-well plate. Hemoglobin release was determined at OD 415 nm using a microplate reader. The percent hemolysis was determined using the following formula:

$$\text{Percent Hemolysis} = 100 \times \frac{(\text{OD patient sample-OD blank})}{(\text{OD human serum control-OD blank})}$$

The graph of the pharmacodynamics (Figure 8), the study of the physiological effects, shows the percentage of serum hemolytic activity (i.e, the percentage of cell lysis) over time. Cell lysis was dramatically reduced in the majority of the patients to below 20% of normal serum complement activity while under eculizumab treatment. Two patients exhibited a breakthrough in complement activity, but complement blockade was permanently restored by reducing the dosing interval to 12 days (See, Figure 4).

Improvement of quality of life issues was also evaluated using the European Organization for Research and Treatment of Cancer Core (http://www.eortc.be) questionnaires ("EORTC QLC-C30"). Each of the participating patients completed the QLC-30 questionnaire before and during the eculizumab therapy. Overall improvements were observed in global health status, physical functioning, role functioning, emotional functioning, cognitive functioning, fatigue, pain, dyspnea and insomnia. (See Figure 9).

Patients in the two year study experienced a reduction in adverse symptoms associated with PNH. For example, as set forth in Figure 10, there was a demonstrated decrease of abdominal pain, dysphagia, and erectile dysfunction after administration of eculizumab in those patients reporting those symptoms before administration of eculizumab.

Although preferred and other embodiments of the invention have been described herein, further embodiments may be perceived by those skilled in the art without departing from the scope of the invention.

The present invention furthermore relates to the following items:

1. A method of treating a hemolytic disease in a subject comprising administering an anti-C5 antibody to a subject having a hemolytic disease, wherein there is a reduction in hemoglobinuria in the subject.
2. A method as in item 1 wherein the anti-C5 antibody is selected from the group consisting of h5G1.1-mAb, h5G1.1-scFv and functional fragments of h5G1.1.
3. A method as in item1 wherein the proportion of type III red blood cells of the subject's total red blood cell content is greater than 10%.
4. A method as in item 1 wherein the proportion of type III red blood cells of the subject's total red blood cell content is greater than 25%.
5. A method as in item 1 wherein the proportion of type III red blood cells of the subject's total red blood cell content is greater than 50%.
6. A method as in item 1 wherein the subject's platelet count is greater than 40,000 per microliter.
7. A method as in item 1 wherein the subject's platelet count is greater than 75,000 per microliter.
8. A method as in item 1 wherein the subject's platelet count is greater than 150,000 per microliter.
9. A method as in item 1 wherein the subject's reticulocyte count is greater than 80 x $10^9$ per liter.
10. A method as in item 1 wherein the subject's reticulocyte count is greater than 120 x $10^9$ per liter.
11. A method as in item 1 wherein the subject's reticulocyte count is greater than 150 x $10^9$ per liter.
12. A method as in item1 wherein said subject has a greater than 40% PNH type III granulocyte clone.
13. A method as in item 1 wherein said subject has an LDH level greater than or equal to 1.5 times the upper limit of a normal LDH level in a person.
14. A method of treating a hemolytic disease in a subject comprising administering to a subject having a hemolytic disease a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors, wherein there is a reduction in hemoglobinuria in the subject.
15. A method as in item 14 wherein the compound is selected from the group consisting of CR1, LEX-CR1, MCP, DAF, CD59, Factor H, cobra venom factor, FUT-175, complestatin, and K76 COOH.
16. A method as in item 14 wherein said subject has a greater than 40% PNH type III granulocyte clone.
17. A method as in item 14 wherein said subject has an LDH level greater than or equal to 1.5 times the upper limit of a normal LDH level in a person.
18. A method of restoring nitric oxide (NO) homeostasis comprising administering an anti-C5 antibody to a subject having a hemolytic disease.
19. A method as in item 18 wherein the anti-C5 antibody is selected from the group consisting of h5G1.1-mAb, h5G1.1-scFv and functional fragments of h5G1.1.
20. A method as in item 18 wherein the proportion of type III red blood cells of the subject's total red blood cell content is greater than 10%.
21. A method as in item 18 wherein the proportion of type III red blood cells of the subject's total red blood cell content is greater than 25%.
22. A method as in item 18 wherein the proportion of type III red blood cells of the subject's total red blood cell content is greater than 50%.
23. A method as in item 18 wherein the subject's platelet count is greater than 40,000 per microliter.
24. A method as in item 18 wherein the subject's platelet count is greater than 75,000 per microliter.
25. A method as in item 18 wherein the subject's platelet count is greater than 150,000 per microliter.
26. A method as in 18 wherein the subject's reticulocyte count is greater than 80 x $10^9$ per liter.
27. A method as in item 18 wherein the subject's reticulocyte count is greater than 120 x $10^9$ per liter.
28. A method as in item 18 wherein the subject's reticulocyte count is greater than 150 x $10^9$ per liter.
29. A method as in item 18 wherein said subject has a greater than 40% PNH type III granulocyte clone.
30. A method as in item 18 wherein said subject has an LDH level greater than or equal to 1.5 times the upper limit of a normal LDH level in a person.
31. A method of restoring nitric oxide (NO) homeostasis comprising administering to a subject having a hemolytic disease a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

32. A method as in item 31 wherein the compound is selected from the group consisting of CR1, LEX-CR1, MCP, DAF, CD59, Factor H, cobra venom factor, FUT-175, complestatin, and K76 COOH.

33. A method as in item 31 wherein said subject has a greater than 40% PNH type III granulocyte clone.

34. A method as in item 31 wherein said subject has an LDH level greater than or equal to 1.5 times the upper limit of a normal LDH level in a person.

35. A method of reducing hemolysis in a subject having a hemolytic disease comprising administering an anti-C5 antibody, wherein hemolysis is reduced as evidenced by a greater than 50% reduction in lactate dehydrogenase (LDH) levels in the subject's bloodstream.

36. A method as in item 35 wherein hemolysis is reduced as evidenced by a greater than 65% reduction in lactate dehydrogenase (LDH) levels in the subject's bloodstream.

37. A method as in item 35 wherein hemolysis is reduced as evidenced by a greater than 80% reduction in lactate dehydrogenase (LDH) levels in the subject's bloodstream.

38. A method as in item 35 wherein the anti-C5 antibody is selected from the group consisting of h5G1.1-mAb, h5G1.1-scFv and functional fragments of h5G1.1.

39. A method as in item 35 wherein the proportion of type III red blood cells of the subject's total red blood cell content is greater than 10%.

40. A method as in item 35 wherein the proportion of type III red blood cells of the subject's total red blood cell content is greater than 25%.

41. A method as in item 35 wherein the proportion of type III red blood cells of the subject's total red blood cell content is greater than 50%.

42. A method as in item 35 wherein the subject's platelet count is greater than 40,000 per microliter.

43. A method as in item 35 wherein the subject's platelet count is greater than 75,000 per microliter.

44. A method as in item 35 wherein the subject's platelet count is greater than 150,000 per microliter.

45. A method as in item 35 wherein the subject's reticulocyte count is greater than 80 x $10^9$ per liter.

46. A method as in item 35 wherein the subject's reticulocyte count is greater than 120 x $10^9$ per liter.

47. A method as in item 35 wherein the subject's reticulocyte count is greater than 150 x $10^9$ per liter.

48. A method as in item 35 wherein said subject has a greater than 40% PNH type III granulocyte clone.

49. A method as in item 35 wherein said subject has an LDH level greater than or equal to 1.5 times the upper limit of a normal LDH level in a person.

50. A method of reducing hemolysis in a subject having a hemolytic disease comprising administering to the subject a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors, wherein hemolysis is reduced as evidenced by a greater than 50% reduction in lactate dehydrogenase (LDH) levels in the subject's bloodstream.

51. A method as in item 50 wherein the compound is selected from the group consisting of CR1, LFX-CR1, MCP, DAF, CD59, Factor H, cobra venom factor, FUT-175, complestatin, and K76 COOH.

52. A method as in item 50 wherein said subject has a greater than 40% PNH type III granulocyte clone.

53. A method as in item 50 wherein said subject has an LDH level greater than or equal to 1.5 times the upper limit of a normal LDH level in a person.

54. A method of reducing hemoglobinuria in a subject comprising administering a compound to the subject, the compound being selected from the group consisting of compounds which bind to one or more complement components, compounds which block the generation of one or more complement components and compounds which block the activity of one or more complement components.

55. A method as in item 54 wherein the step of administering comprises administering an anti-C5 antibody.

56. A method as in item 54 wherein the compound is an anti-C5 antibody selected from the group consisting of h5G1.1-mAb, h5G1.1-scFv and functional fragments of h5G1.1.

57. A method as in item 54 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

58. A method as in item 54 wherein the step of administering comprises administering a compound selected from the group consisting of CR1, LEX-CR1, MCP, DAF, CD59, Factor H, cobra venom factor, FUT-175, complestatin, and K76 COOH.

59. A method as in item 54 wherein the proportion of type III red blood cells of the subject's total red blood cell content is greater than 10%.

60. A method as in item 54 wherein the proportion of type III red blood cells of the subject's total red blood cell content is greater than 25%.

61. A method as in item 54 wherein the proportion of type III red blood cells of the subject's total red blood cell content is greater than 50%.

62. A method as in item 54 wherein the subject's platelet count is greater than 40,000 per microliter.

63. A method as in item 54 wherein the subject's platelet count is greater than 75,000 per microliter.

64. A method as in item 54 wherein the subject's platelet count is greater than 150,000 per microliter.

65. A method as in item 54 wherein the subject's reticulocyte count is greater than 80 x $10^9$ per liter.

66. A method as in item 54 wherein the subject's reticulocyte count is greater than 120 x $10^9$ per liter.

67. A method as in item 54 wherein the subject's reticulocyte count is greater than 150 x $10^9$ per liter.

68. A method as in item 54 wherein said subject has a greater than 40% PNH type III granulocyte clone.

69. A method as in item 54 wherein said subject has an LDH level greater than or equal to 1.5 times the upper limit of a normal LDH level in a person.

70. A method of reducing dysphagia in a subject comprising administering a compound to the subject, the compound being selected from the group consisting of compounds which bind to one or more complement components and compounds which block the generation of one or more complement components.

71. A method as in item 70 wherein the step of administering comprises administering an anti-C5 antibody.

72. A method as in item 70 wherein the compound is an anti-C5 antibody selected from the group consisting of h5G1.1-mAb, h5G1.1-scFv and functional fragments of h5G1.1.

73. A method as in item 70 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

74. A method as in item 70 wherein the step of administering comprises administering a compound selected from the group consisting of CR1, LEX-CR1, MCP, DAF, CD59, Factor H, cobra venom factor, FUT-175, complestatin, and K76 COOH.

75. A method as in item 70 wherein the proportion of type III red blood cells of the subject's total red blood cell content is greater than 10%.

76. A method as in item 70 wherein the proportion of type III red blood cells of the subject's total red blood cell content is greater than 25%.

77. A method as in item 70 wherein the proportion of type III red blood cells of the subject's total red blood cell content is greater than 50%.

78. A method as in item 70 wherein the subject's platelet count is greater than 40,000 per microliter.

79. A method as in item 70 wherein the subject's platelet count is greater than 75,000 per microliter.

80. A method as in item 70 wherein the subject's platelet count is greater than 150,000 per microliter.

81. A method as in item 70 wherein the subject's reticulocyte count is greater than 80 x $10^9$ per liter.

82. A method as in item 70 wherein the subject's reticulocyte count is greater than 120 x $10^9$ per liter.

83. A method as in item 70 wherein the subject's reticulocyte count is greater than 150 x $10^9$ per liter.

84. A method as in item 70 wherein said subject has a greater than 40% PNH type III granulocyte clone.

85. A method as in item 70 wherein said subject has an LDH level greater than or equal to 1.5 times the upper limit of a normal LDH level in a person.

86. A method of reducing erectile dysfunction in a subject comprising administering a compound to the subject, the compound being selected from the group consisting of compounds which bind to one or more complement components and compounds which block the generation of one or more complement components.

87. A method as in item 86 wherein the step of administering comprises administering an anti-C5 antibody.

88. A method as in item 86 wherein the compound is an anti-C5 antibody selected from the group consisting of h5G1.1-mAb, h5G1.1-scFv and functional fragments of h5G1.1

89. A method as in item 86 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

90. A method as in item 86 wherein the step of administering comprises administering a compound selected from the group consisting of CR1, LEX-CR1, MCP, DAF, CD59, Factor H, cobra venom factor, FUT-175, complestatin, and K76 COOH.

91. A method as in item 86 wherein the proportion of type III red blood cells of the subject's total red blood cell content is greater than 10%.

92. A method as in item 86 wherein the proportion of type III red blood cells of the subject's total red blood cell content is greater than 25%.
93. A method as in item 86 wherein the proportion of type III red blood cells of the subject's total red blood cell content is greater than 50%.
94. A method as in 86 wherein the subject's platelet count is greater than 40,000 per microliter.
95. A method as in item 86 wherein the subject's platelet count is greater than 75,000 per microliter.
96. A method as in item 86 wherein the subject's platelet count is greater than 150,000 per microliter.
97. A method as in item 86 wherein the subject's reticulocyte count is greater than 80 x $10^9$ per liter.
98. A method as in item 86 wherein the subject's reticulocyte count is greater than 120 x $10^9$ per liter.
99. A method as in item 86 wherein the subject's reticulocyte count is greater than 150 x $10^9$ per liter.
100. A method as in item 85 wherein said subject has a greater than 40% PNH type III granulocyte clone.
101. A method as in item 86 wherein said subject has an LDH level greater than or equal to 1.5 times the upper limit of a normal LDH level in a person.
102. A method of reducing thrombosis in a subject comprising administering a compound to a subject, the compound being selected from the group consisting of compounds which bind to one or more complement components, compounds which block the generation of one or more complement components and compounds which block the activity of one or more complement components.
103. A method as in item 102 wherein the step of administering comprises administering an anti-C5 antibody.
104. A method as in item 102 wherein the compound is an anti-C5 antibody selected from the group consisting of h5G1.1-mAb, h5G1.1-scFv and functional fragments of h5G1.1.
105. A method as in item 102 wherein the step of administering comprises administering an anti-C5a receptor antibody.
106. A method as in item 102 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.
107. A method as in item 102 wherein the step of administering comprises administering a compound selected from the group consisting of CR1, LEX-CR1, MCP, DAF, CD59, Factor H, cobra venom factor, FUT-175, complestatin, and K76 COOH.
108. A method as in item 102 wherein the proportion of type III red blood cells of the subject's total red blood cell content is greater than 10%.
109. A method as in item 102 wherein the proportion of type III red blood cells of the subject's total red blood cell content is greater than 25%.
110. A method as in item 102 wherein the proportion of type III red blood cells of the subject's total red blood cell content is greater than 50%.
111. A method as in item 102 wherein the subject's platelet count is greater than 40,000 per microliter.
112. A method as in item 102 wherein the subject's platelet count is greater than 75,000 per microliter.
113. A method as in item 102 wherein the subject's platelet count is greater than 150,000 per microliter.
114. A method as in item 102 wherein the subject's reticulocyte count is greater than 80 x $10^9$ per liter.
115. A method as in item 102 wherein the subject's reticulocyte count is greater than 120 x $10^9$ per liter.
116. A method as in item 102 wherein the subject's reticulocyte count is greater than 150 x $10^9$ per liter.
117. A method as in item 102 wherein said subject has a greater than 40% PNH type III granulocyte clone.
118. A method as in item 102 wherein said subject has an LDH level greater than or equal to 1.5 times the upper limit of a normal LDH level in a person.
119. A method of increasing proportion of type III red blood cells of a subject's total red blood cell content comprising administering a compound to the subject, the compound being selected from the group consisting of compounds which bind to one or more complement components, compounds which block the generation of one or more complement components and compounds which block the activity of one or more complement components.
120. A method as in item 119 wherein the step of administering comprises administering an anti-C5 antibody.
121. A method as in item 119 wherein the compound is an anti-C5 antibody selected from the group consisting of h5G1.1-mAb, h5G1.1-scFv and functional fragments of h5G1.1.
122. A method as in item 119 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.
123. A method as in item 119 wherein the step of administering comprises administering a compound selected from the group consisting of CR1, LEX-CR1, MCP, DAF, CD59, Factor H, cobra venom factor, FUT-175, complestatin, and K76 COOH.

124. A method as in item 119 wherein the amount of PNH type III red blood cells is greater than about 10% of the subject's total red blood cell count.
125. A method as in item 119 wherein the amount of PNH type III red blood cells is greater than about 25% of the subject's total red blood cell count.
126. A method as in item 119 wherein the amount of PNH type III red blood cells is greater than about 50% of the subject's total red blood cell count.
127. A method as in item 119 wherein the amount of PNH type III red blood cells is greater than about 75% of the subject's total red blood cell count.
128. A method as in item 119 wherein the subject's platelet count is greater than 40,000 per microliter.
129. A method as in item 119 wherein the subject's platelet count is greater than 75,000 per microliter.
130. A method as in item 119 wherein the subject's platelet count is greater than 150,000 per microliter.
131. A method as in item 119 wherein the subject's reticulocyte count is greater than 80 x $10^9$ per liter.
132. A method as in item 119 wherein the subject's reticulocyte count is greater than 120 x $10^9$ per liter.
133. A method as in item 119 wherein the subject's reticulocyte count is greater than 150 x $10^9$ per liter.
134. A method as in item 119 wherein said subject has a greater than 40% PNH type III granulocyte clone.
135. A method as in item 119 wherein said subject has an LDH level greater than or equal to 1.5 times the upper limit of a normal LDH level in a person.
136. A method of reducing hemolysis in a subject comprising administering a compound to the subject, the compound being selected from the group consisting of compounds which bind to one or more complement components, compounds which block the generation of one or more complement components and compounds which block the activity of one or more complement components.
137. A method as in item 136 wherein the step of administering comprises administering an anti-C5 antibody.
138. A method as in item 136 wherein the compound is an anti-C5 antibody selected from the group consisting of h5G1.1-mAb, h5G1.1-scFv and functional fragments of h5G1.1.
139. A method as in item 136 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.
140. A method as in item 136 wherein the step of administering comprises administering a compound selected from the group consisting of CR1, LEX-CR1, MCP, DAF, CD59, Factor H, cobra venom factor, FUT-175, complestatin, and K76 COOH.
141. A method as in item 136 wherein the proportion of type III red blood cells of the subject's total red blood cell content is greater than 10%.
142. A method as in item 136 wherein the proportion of type III red blood cells of the subject's total red blood cell content is greater than 25%.
143. A method as in item 136 wherein the proportion of type III red blood cells of the subject's total red blood cell content is greater than 50%.
144. A method as in item 136 wherein the subject's platelet count is greater than 40,000 per microliter.
145. A method as in item 136 wherein the subject's platelet count is greater than 75,000 per microliter.
146. A method as in item 136 wherein the subject's platelet count is greater than 150,000 per microliter.
147. A method as in item 136 wherein the subject's reticulocyte count is greater than 80 x $10^9$ per liter.
148. A method as in item 136 wherein the subject's reticulocyte count is greater than 120 x $10^9$ per liter.
149. A method as in item 136 wherein the subject's reticulocyte count is greater than 150 x $10^9$ per liter.
150. A method as in item 136 wherein said subject has a greater than 40% PNH type III granulocyte clone.
151. A method as in item 136 wherein said subject has an LDH level greater than or equal to 1.5 times the upper limit of a normal LDH level in a person.
152. A method of treating a nitric oxide (NO) deficiency in a subject afflicted with a hemolytic disease comprising administering a compound to the subject, the compound being selected from the group consisting of compounds which bind to one or more complement components, compounds which block the generation of one or more complement components and compounds which block the activity of one or more complement components.
153. A method as in item 152 wherein the step of administering comprises administering an anti-C5 antibody.
154. A method as in item 152 wherein the compound is an anti-C5 antibody selected from the group consisting of h5G1.1-mAb, h5G1.1-scFv and functional fragments of h5G1.1.
155. A method as in item 152 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.
156. A method as in item 152 wherein the step of administering comprises administering a compound selected from the group consisting of CR1, LEX-CR1, MCP, DAF, CD59, Factor H, cobra venom factor, FUT-175, complestatin, and K76 COOH.

157. A method as in item 152 wherein the proportion of type III red blood cells of the subject's total red blood cell content is greater than 10%.

158. A method as in item 152 wherein the proportion of type III red blood cells of the subject's total red blood cell content is greater than 25%.

159. A method as in item 152 wherein the proportion of type III red blood cells of the subject's total red blood cell content is greater than 50%.

160. A method as in item 152 wherein the subject's platelet count is greater than 40,000 per microliter.

161. A method as in item 152 wherein the subject's platelet count is greater than 75,000 per microliter.

162. A method as in item 152 wherein the subject's platelet count is greater than 150,000 per microliter.

163. A method as in item 152 wherein the subject's reticulocyte count is greater than 80 x $10^9$ per liter.

164. A method as in item 152 wherein the subject's reticulocyte count is greater than 120 x $10^9$ per liter.

165. A method as in item 152 wherein the subject's reticulocyte count is greater than 150 x $10^9$ per liter.

166. A method as in item 152 wherein said subject has a greater than 40% PNH type III granulocyte clone.

167. A method as in item 152 wherein said subject has an LDH level greater than or equal to 1.5 times the upper limit of a normal LDH level in a person.

168. A method of rendering a subject afflicted with a hemolytic disease transfusion independent comprising administering a compound to the subject, the compound being selected from the group consisting of compounds which bind to one or more complement components, compounds which block the generation of one or more complement components and compounds which block the activity of one or more complement components.

169. A method as in item 168 wherein the step of administering comprises administering an anti-C5 antibody.

170. A method as in item 168 wherein the compound is an anti-C5 antibody selected from the group consisting of h5G1.1-mAb, h5G1.1-scFv and functional fragments of h5G1.1.

171. A method as in item 168 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

172. A method as in item 168 wherein the step of administering comprises administering a compound selected from the group consisting of CR1, LEX-CR1, MCP, DAF, CD59, Factor H, cobra venom factor, FUT-175, complestatin, and K76 COOH.

173. A method as in item 168 further comprising the step of administering one or more compounds that increase hematopoiesis in combination with said compound.

174. A method as in item 173 wherein the one or more compounds that increase hematopoiesis is selected from the group consisting of steroids, immunosuppressants, anti-coagulants, folic acid, iron, erythropoietin (EPO), antithymocyte globulin (ATG) and antilymphocyte globulin (ALG).

175. A method as in item 173 wherein EPO is administered in combination with an anti-C5 antibody selected from the group consisting of h5G1.1-mAb, h5G1.1-scFv and functional fragments of h5G1.1.

176. A method as in item 168 wherein the subject is transfusion independent for over six months from the first administration of said compound.

177. A method as in item 168 wherein the subject is transfusion independent for over twelve months from the first administration of said compound.

178. A method as in item 168 wherein the subject is transfusion independent for over two years from the first administration of said compound.

179. A method as in item 168 wherein the proportion of type III red blood cells of the subject's total red blood cell content is greater than 10%.

180. A method as in item 168 wherein the proportion of type III red blood cells of the subject's total red blood cell content is greater than 25%.

181. A method as in item 158 wherein the proportion of type III red blood cells of the subject's total red blood cell content is greater than 50%.

182. A method as in item 168 wherein the subject's platelet count is greater than 40,000 per microliter.

183. A method as in item 168 wherein the subject's platelet count is greater than 75,000 per microliter.

184. A method as in item 168 wherein the subject's platelet count is greater than 150,000 per microliter.

185. A method as in item 168 wherein the subject's reticulocyte count is greater than 80 x $10^9$ per liter.

186. A method as in item 168 wherein the subject's reticulocyte count is greater than 120 x $10^9$ per liter.

187. A method as in item 168 wherein the subject's reticulocyte count is greater than 150 x $10^9$ per liter.

188. A method as in item 168 wherein said subject has a greater than 40% PNH type III granulocyte clone.

189. A method as in item 168 wherein said subject has an LDH level greater than or equal to 1.5 times the upper limit of a normal LDH level in a person.

190. A method of treating a subject afflicted with a hemolytic disease comprising administering: 1) one or more compounds selected from the group consisting of compounds which bind to one or more complement components, compounds which block the generation of one or more complement components and compounds which block the activity of one or more complement components; in combination with 2) one or more compounds that increase hematopoiesis.

191. A method as in item 190 wherein the one or more compounds that increase hematopoiesis are selected from the group consisting of steroids, immunosuppressants, anti-coagulants, folic acid, iron, erythropoietin (EPO), antithymocyte globulin (ATG) and antilymphocyte globulin (ALG).

192. A method as in item 190 wherein EPO is administered in combination with an anti-C5 antibody selected from the group consisting of h5G1.1-mAb, h5G1.1-scFv and functional fragments of h5G1.1.

193. A method as in item 190 wherein EPO is administered in combination with a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

194. A method as in item 190 wherein EPO is administered in combination with a compound selected from the group consisting of CR1, LEX-GR1, MCP, DAF, CD59, Factor H, cobra venom factor, FUT-175, complestatin, and K76 COOH.

195. A method as in item 190 wherein the proportion of type III red blood cells of the subject's total red blood cell content is greater than 10%.

196. A method as in item 190 wherein the proportion of type III red blood cells of the subject's total red blood cell content is greater than 25%.

197. A method as in item 190 wherein the proportion of type III red blood cells of the subject's total red blood cell content is greater than 50%.

198. A method as in item 190 wherein the subject's platelet count is greater than 40,000 per microliter.

199. A method as in item 190 wherein the subject's platelet count is greater than 75,000 per microliter.

200. A method as in item 190 wherein the subject's platelet count is greater than 150,000 per microliter.

201. A method as in item 190 wherein the subject's reticulocyte count is greater than 80 x $10^9$ per liter.

202. A method as in item 190 wherein the subject's reticulocyte count is greater than 120 x $10^9$ per liter.

203. A method as in item 190 wherein the subject's reticulocyte count is greater than 150 x $10^9$ per liter.

204. A method as in item 190 wherein said subject has a greater than 40% PNH type III granulocyte clone.

205. A method as in item 190 wherein said subject has an LDH level greater than or equal to 1.5 times the upper limit of a normal LDH level in a person.

206. A method of reducing hemolysis in a subject having a hemolytic disease comprising administering an anti-C5 antibody, wherein hemolysis is reduced as evidenced by a reduction in lactate dehydrogenase (LDH) levels in the subject's bloodstream to within 20% of the upper limit of normal.

207. A method as in item 206 wherein the anti-C5 antibody is selected from the group consisting of h5G1.1-mAb, h5G1.1-scFv and functional fragments of h5G1.1.

208. A method as in item 206 wherein the proportion of type III red blood cells of the subject's total red blood cell content is greater than 10%.

209. A method as in item 206 wherein the proportion of type III red blood cells of the subject's total red blood cell content is greater than 25%.

210. A method as in item 206 wherein the proportion of type III red blood cells of the subject's total red blood cell content is greater than 50%.

211. A method as in item 206 wherein the subject's platelet count is greater than 40,000 per microliter.

212. A method as in item 206 wherein the subject's platelet count is greater than 75,000 per microliter.

213. A method as in item 206 wherein the subject's platelet count is greater than 150,000 per microliter.

214. A method as in item 206 wherein the subject's reticulocyte count is greater than 80 x $10^9$ per liter.

215. A method as in item 206 wherein the subject's reticulocyte count is greater than 120 x $10^9$ per liter.

216. A method as in item 206 wherein the subject's reticulocyte count is greater than 150 x $10^9$ per liter.

217. A method as in item 206 wherein said subject has a greater than 40% PNH type III granulocyte clone.

218. A method as in item 206 wherein said subject has an LDH level greater than or equal to 1.5 times the upper limit of a normal LDH level in a person.

219. A method of reducing hemolysis in a subject having a hemolytic disease comprising administering to the subject a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors, wherein hemolysis is reduced as evidenced by a reduction in lactate dehydrogenase (LDH) levels in the subject's bloodstream to within 20% of the upper limit of normal.

220. A method as in item 219 wherein the compound is selected from the group consisting of CR1, LEX-CR1, MCP, DAF, CD59, Factor H, cobra venom factor, FUT-175, complestatin, and K76 COOH.

221. A method as in item 219 wherein said subject has a greater than 40% PNH type III granulocyte clone.

222. A method as in item 219 wherein said subject has an LDH level greater than or equal to 1.5 times the upper limit of a normal LDH level in a person.

223. A method of reducing hemolysis in a subject having a hemolytic disease comprising administering an anti-C5 antibody, wherein serum complement hemolytic activity is reduced at least 80% as evidenced by a serum hemolytic assay.

224. A method as in item 223 wherein the anti-C5 antibody is selected from the group consisting of h5G1.1-mAb, h5G1.1-scFv and functional fragments of h5G1.1.

225. A method as in item 223 wherein the proportion of type III red blood cells of the subject's total red blood cell content is greater than 10%.

226. A method as in item 223 wherein the proportion of type III red blood cells of the subject's total red blood cell content is greater than 25%.

227. A method as in item 223 wherein the proportion of type III red blood cells of the subject's total red blood cell content is greater than 50%.

228. A method as in item 223 wherein the subject's platelet count is greater than 40,000 per microliter.

229. A method as in item 23 wherein the subject's platelet count is greater than 75,000 per microliter.

230. A method as in item 223 wherein the subject's platelet count is greater than 150,000 per microliter.

231. A method as in item 223 wherein the subject's reticulocyte count is greater than 80 x $10^9$ per liter.

232. A method as in item 223 wherein the subject's reticulocyte count is greater than 120 x $10^9$ per liter.

233. A method as in item 223 wherein the subject's reticulocyte count is greater than 150 x $10^9$ per liter.

234. A method as in item 223 wherein said subject has a greater than 40% PNH type III granulocyte clone.

235. A method as in item 223 wherein said subject has an LDH level greater than or equal to 1.5 times the upper limit of a normal LDH level in a person.

236. A method of reducing hemolysis in a subject having a hemolytic disease comprising administering to the subject a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors, wherein serum complement hemolytic activity is reduced at least 80% as evidenced by a serum hemolytic assay.

237. A method as in item 236 wherein the compound is selected from the group consisting of CR1, LEX-CR1, MCP, DAF, CD59, Factor H, cobra venom factor, FUT-175, complestatin, and K76 COOH.

238. A method as in item 236 wherein said subject has a greater than 40% PNH type III granulocyte clone.

239. A method as in item 236 wherein said subject has an LDH level greater than or equal to 1.5 times the upper limit of a normal LDH level in a person.

240. A method of reducing abdominal pain in a subject comprising administering a compound to the subject, the compound being selected from the group consisting of compounds which bind to one or more complement components, compounds which block the generation of one or more complement components and compounds which block the activity of one or more complement components.

241. A method as in item 240 wherein the step of administering comprises administering an anti-C5 antibody.

242. A method as in item 240 wherein the compound is an anti-C5 antibody selected from the group consisting of h5G1.1-mAb, h5G1.1-scFv and functional fragments of h5G1.1.

243. A method as in item 240 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.

244. A method as in item 240 wherein the step of administering comprises administering a compound selected from the group consisting of CR1, LEX-CR1, MCP, DAF, CD59, Factor H, cobra venom factor, FUT-175, complestatin, and K76 COOH.

245. A method as in item 240 wherein the proportion of type III red blood cells of the subject's total red blood cell content is greater than 10%.

246. A method as in item 240 wherein the proportion of type III red blood cells of the subject's total red blood cell content is greater than 25%.

247. A method as in item 240 wherein the proportion of type III red blood cells of the subject's total red blood cell content is greater than 50%.

248. A method as in item 240 wherein the subject's platelet count is greater than 40,000 per microliter.

249. A method as in item 240 wherein the subject's platelet count is greater than 75,000 per microliter.
250. A method as in item 240 wherein the subject's platelet count is greater than 150,000 per microliter.
251. A method as in item 240 wherein the subject's reticulocyte count is greater than 80 x $10^9$ per liter.
252. A method as in item 240 wherein the subject's reticulocyte count is greater than 120 x $10^9$ per liter.
253. A method as in item 240 wherein the subject's reticulocyte count is greater than 150 x $10^9$ per liter.
254. A method as in item 240 wherein said subject has a greater than 40% PNH type III granulocyte clone.
255. A method as in item 240 wherein said subject has an LDH level greater than or equal to 1.5 times the upper limit of a normal LDH level in a person.
256. A method of decreasing the frequency of transfusions required by a subject afflicted with a hemolytic disease comprising administering a compound to the subject, the compound being selected from the group consisting of compounds which bind to one or more complement components, compounds which block the generation of one or more complement components and compounds which block the activity of one or more complement components.)
257. The method of item 256 wherein the frequency of transfusions is decreased by about 50%.
258. The method of item 256 wherein the frequency of transfusions is decreased by about 70%.
259. The method of item 256 wherein the frequency of transfusions is decreased by about 90%.
260. A method as in item 256 wherein the step of administering comprises administering an anti-C5 antibody.
261. A method as in item 256 wherein the step of administering comprises administering an anti-C5 antibody selected from the group consisting of h5G1.1-mAb; h5G1.1-scFv and functional fragments of h5G1.1.
262. A method as in item 256 wherein the step of administering comprises administering a compound selected from the group consisting of antibodies, soluble complement inhibitory compounds, proteins, protein fragments, peptides, small molecules, RNA aptamers, L-RNA aptamers, spiegelmers, antisense compounds, serine protease inhibitors, double stranded RNA, small interfering RNA, locked nucleic acid inhibitors, and peptide nucleic acid inhibitors.
263. A method as in item 256 wherein the step of administering comprises administering a compound selected from the group consisting of CR1, LEX-CR1, MCP, DAF, CD59, Factor H, cobra venom factor, FUT-175, complestatin, and K76 COOH.
264. A method as in item 256 further comprising the step of administering one or more compounds that increase hematopoiesis in combination with said compound.
265. A method as in item 264 wherein the one or more compounds that increase hematopoiesis is selected from the group consisting of steroids, immunosuppressants, anti-coagulants, folic acid, iron, erythropoietin (EPO), antithymocyte globulin (ATG) and antilymphocyte globulin (ALG).
266. A method as in item 264 wherein EPO is administered in combination with an anti-C5 antibody selected from the group consisting of h5G1.1-mAb, h5G1.1-scFv and functional fragments of h5G1.1.
267. A method as in item 256 wherein the proportion of type III red blood cells of the subject's total red blood cell content is greater than 10%.
268. A method as in item 256 wherein the proportion of type III red blood cells of the subject's total red blood cell content is greater than 25%.
269. A method as in item 256 wherein the proportion of type III red blood cells of the subject's total red blood cell content is greater than 50%.
270. A method as in item 256 wherein the subject's platelet count is greater than 40,000 per microliter.
271. A method as in item 256 wherein the subject's platelet count is greater than 75,000 per microliter.
272. A method as in item 256 wherein the subject's platelet count is greater than 150,000 per microliter.
273. A method as in item 256 wherein the subject's reticulocyte count is greater than 80 x $10^9$ per liter.
274. A method as in item 255 wherein the subject's reticulocyte count is greater than 120 x $10^9$ per liter.
275. A method as in item 256 wherein the subject's reticulocyte count is greater than 150 x $10^9$ per liter.
276. A method as in item 256 wherein said subject has a greater than 40% PNH type III granulocyte clone.
277. A method as in item 256 wherein said subject has an LDH level greater than or equal to 1.5 times the upper limit of a normal LDH level in a person.

## Claims

1. An anti-C5 antibody that reduces the conversion of complement component C5 into complement components C5a and C5b for use in treating a patient afflicted with paroxysmal nocturnal hemoglobinuria (PNH), wherein the patient is at risk of life-threatening hemolysis under a prior dosing schedule of the anti-C5 antibody comprising: (i) 600 mg every 7 days for the first 4 weeks, (ii) 900 mg for the fifth dose 7 days after (i), and (iii) 900 mg every 14 days thereafter.

2. The anti-C5 antibody for use according to claim 1, wherein the anti-C5 antibody is a whole antibody or a C5-binding fragment thereof.

**3.** The anti-C5 antibody for use according to claim 1 or 2, wherein the anti-C5 antibody is eculizumab.

**4.** The anti-C5 antibody for use according to claim 1 or 2, wherein the anti-C5 antibody is (i) eculizumab and (ii) to be administered to the PNH patient at a dose of 900 mg every 12 days.

**5.** The anti-C5 antibody for use according to claim 1 or 2, wherein the anti-C5 antibody is pexelizumab.

**6.** The anti-C5 antibody for use according to any one of claims 1-5, wherein the PNH patient is **characterized by** one or more characteristics selected from the group consisting of:

(a) the proportion of type III red blood cells of the patient's total red blood cell count is greater than 10%;
(b) the proportion of type III red blood cells of the patient's total red blood cell content is greater than 25%;
(c) the proportion of type III red blood cells of the patient's total red blood cell content is greater than 50%;
(d) the patient's platelet count is greater than 40,000 per microliter;
(e) the patient's platelet count is greater than 75,000 per microliter;
(f) the patient's platelet count is greater than 150,000 per microliter;
(g) the patient's reticulocyte count is greater than 80 x $10^9$ per liter;
(h) the patient's reticulocyte count is greater than 120 x $10^9$ per liter;
(i) the patient's reticulocyte count is greater than 150 x $10^9$ per liter;
(j) the patient has a greater than 40% type III granulocyte clone; and
(k) the patient has a lactate dehydrogenase (LDH) level greater than or equal to 1.5 times the upper limit of a normal LDH level in a person.

**7.** The anti-C5 antibody for use according to any one of claims 1-6, wherein the PNH patient has experienced life-threatening hemolysis under the prior dosing schedule.

**8.** The anti-C5 antibody for use according to any one of claims 1-7, wherein thrombosis associated with hemolysis threatens the life of the PNH patient.

**9.** The anti-C5 antibody for use according to any one of claims 1-8, wherein hemolytic anemia associated with hemolysis threatens the life of the PNH patient.

Figure 1A.
Biochemical Parameters of Hemolysis During Eculizumab Treatment

| Biochemical Marker | Normal range | Time of Analysis | | | p-value[a] |
|---|---|---|---|---|---|
| | | Pre-study[b] | 12 weeks | 64 weeks | |
| LDH (IU/L) | 150 - 480 | 3110.7 +/- 598.4 | 594.0 +/- 31.7 | 622.4 +/-41.1 | 0.002 |
| AST (IU/L) | 10 - 40 | 76.2 +/- 16.0 | 26.2 +/- 2.3 | 30.1 +/- 3.2 | 0.02 |
| Haptoglobin (g/L) | 0.5 - 2 | <0.06 | <0.06 | 0.14 +/- 0.07[c] | N.S.[d] |
| Hemoglobin (g/dL) | 11.5 - 18 | 10.0 +/- 0.4 | 10.3 +/- 0.4 | 10.4 +/- 0.4 | N.S. |
| Bilirubin | 3 - 15 | 25.9 +/- 4.3 | 28.2 +/- 4.4 | 28.7 +/- 4.0 | N.S. |
| Reticulocytes ($x10^{-3}/mm^3$) | 20 - 80 | 161.4 +/- 25.9 | 191.2 +/- 23.6 | 189.6 +/- 21.8 | N.S. |

[a]from comparisons of mean change from pre-study to 64 weeks
[b]values represent means during 52 week period prior to treatment except for AST which represents the baseline mean
[c]10 of 11 patients were below the detectable limit of haptoglobin (<0.06 g/L); 1 patient had a value of 0.69 g/L.
[d]not significant

# Figure 1b

## Figure 1b: LDH values during eculizumab therapy

- Intravascular hemolysis is well controlled by eculizumab as indicated by the marked and sustained reduction in LDH (mean LDH levels decreased from 3111 +/- 598 U/L over the 12 months prior to treatment to 634 +/- 34 U/L during treatment (p=0.002).
- Patient 010-010 *and* 011-001 *had transient breakthroughs early in the treatment* phase which were completely resolved by adjusting the dosing interval.
- Patient 010-002 discontinued eculizumab near week 103 and LDH levels quickly increased.
- *These sharp increases in LDH levels demonstrate the causal relationship* between complement activity and hemolysis.

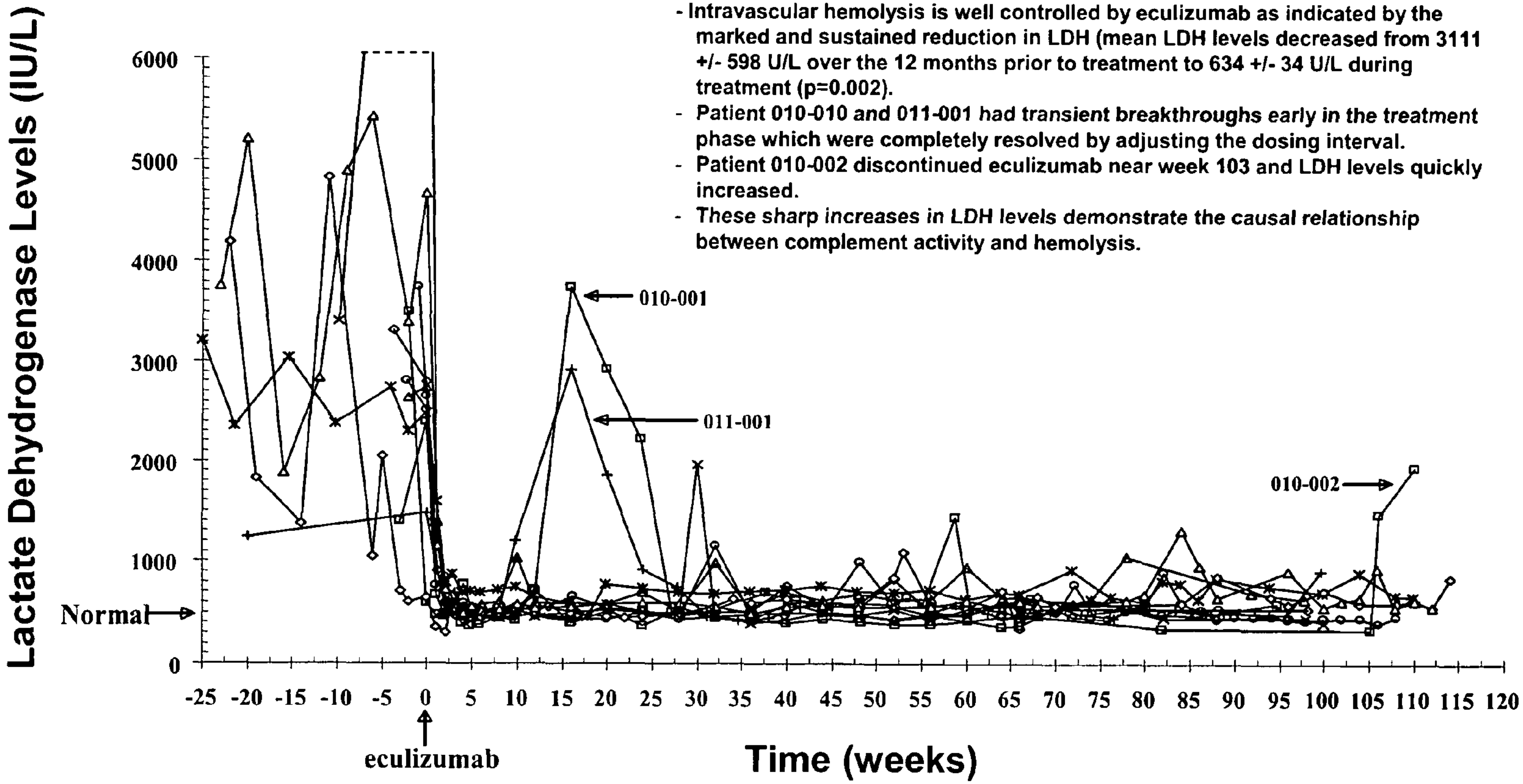

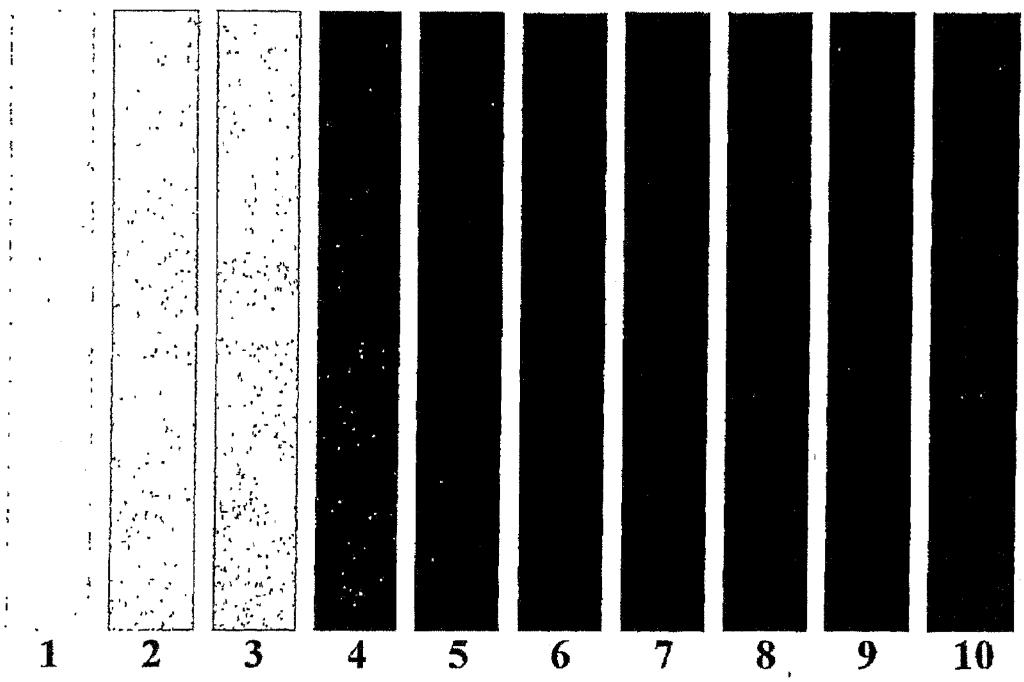


Figure 2

Figure 3.
Effect of Eculizumab on Paroxysm Rate (n=8)

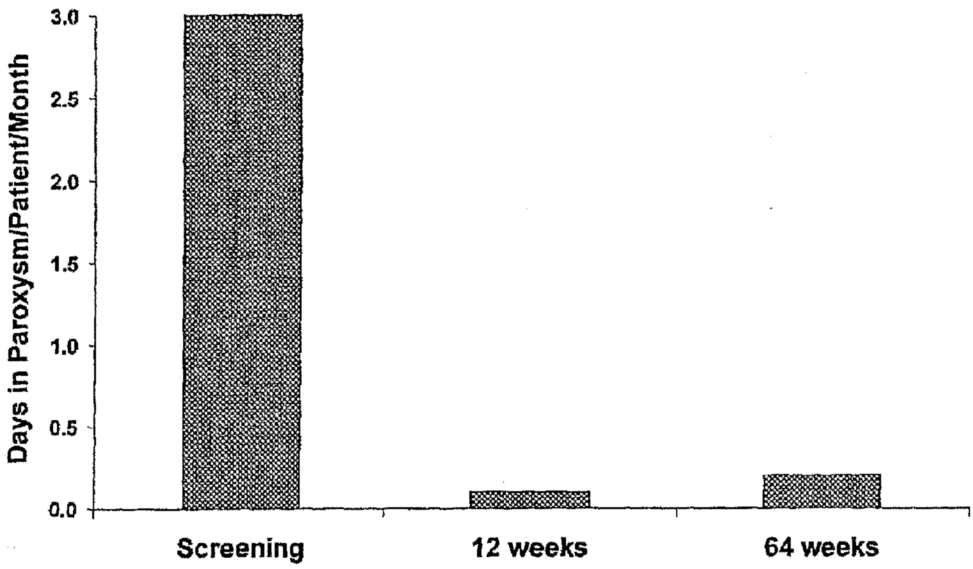


Bars represent the paroxysm rates (number of paroxysms per patient per month) during the screening period (pre-eculizumab treatment), and during the first 12 weeks and the entire 64 weeks of eculizumab treatment. Three patients were not included in the analysis as either pretreatment urine scores were inadvertently not collected (2 patients) or an iron chelating agent that resulted in artificially colored urine was administered during the extension study (1 patient).

# Figure 4.
## Analysis of Complement Breakthrough

**Early Morning Urine**

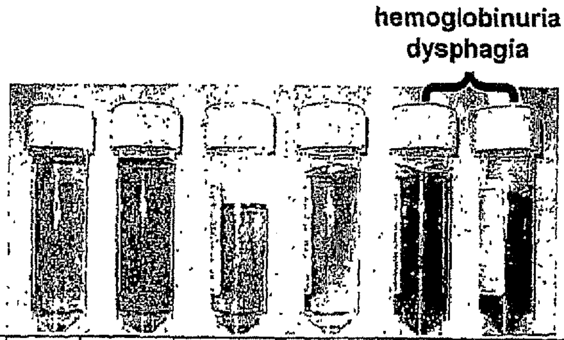


| Visit | 4 | | | | | | | 5 | | | 9 |
|---|---|---|---|---|---|---|---|---|---|---|---|
| Day | 0* | 1-8 | 9 | 10 | 11 | 12 | 13 | 0* | 1-2 | | 0* |
| Urine | 10 | 2-3 | 3 | 3 | 3 | 3 | 9 | 10 | 3 | | 2 |
| LDH | 2624 | – | 784 | – | – | 697 | 1687 | 2917 | – | | 495 |
| AST | 119 | – | 38 | – | – | 31 | 87 | – | – | | – |
| PK | 28 | – | 51 | – | – | 35 | 31 | 23 | – | | 51 |
| PD | 72 | – | 1 | – | – | 2 | 56 | 56 | – | | 2 |

*Dose of eculizumab

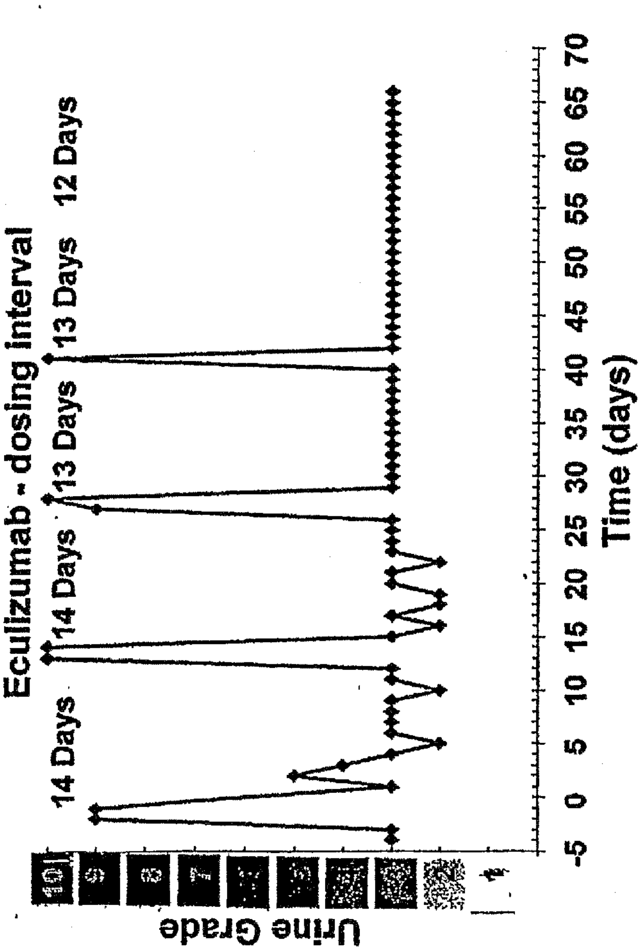


Fig 5. Patient 010-010 Urine Grade vs. Time

# Figure 6

Units Transfused Pre- and Post-Eculizumab in (a) Cytopenic Patients and (b) Non-Cytopenic Patients

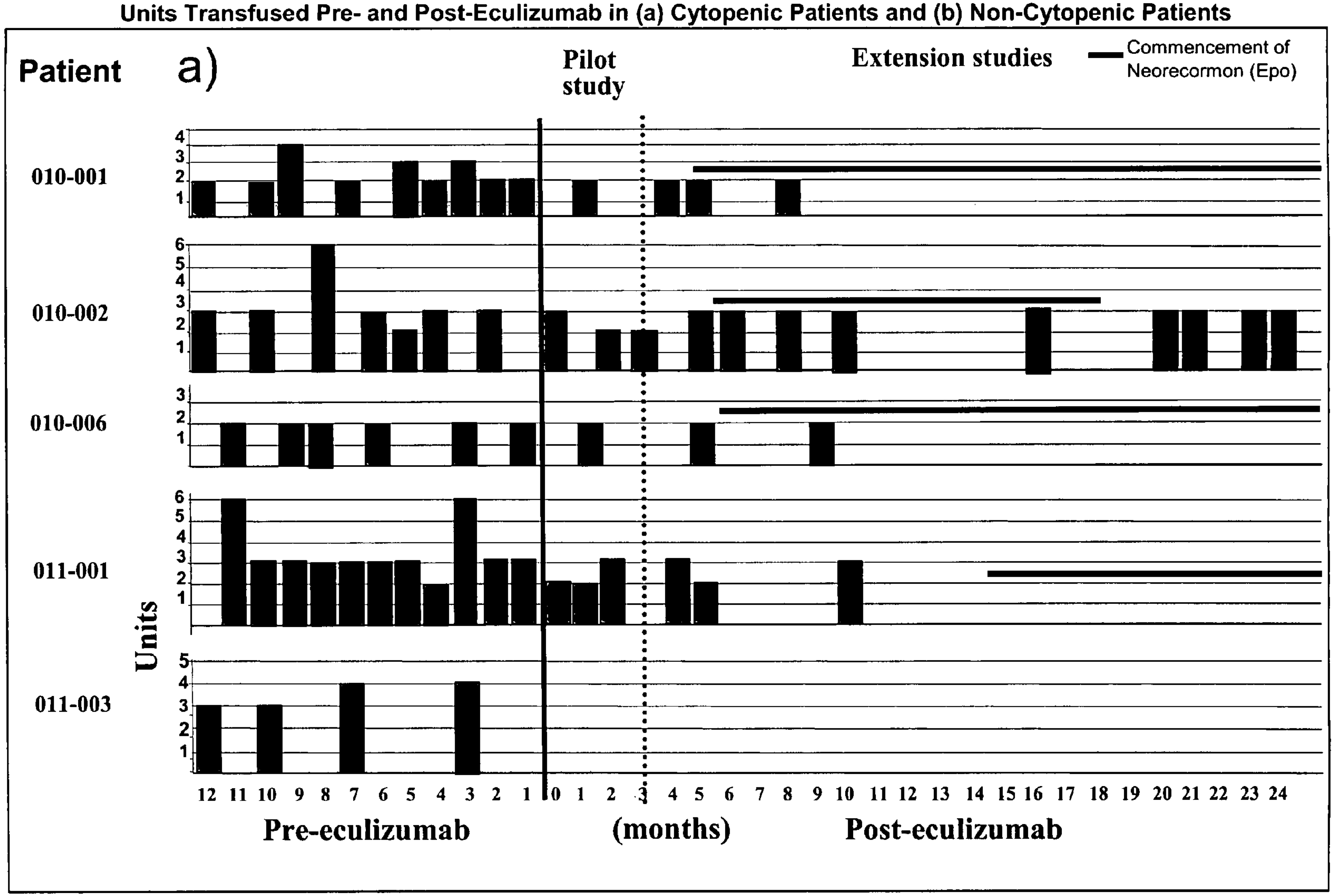

Figure 6 (continued)

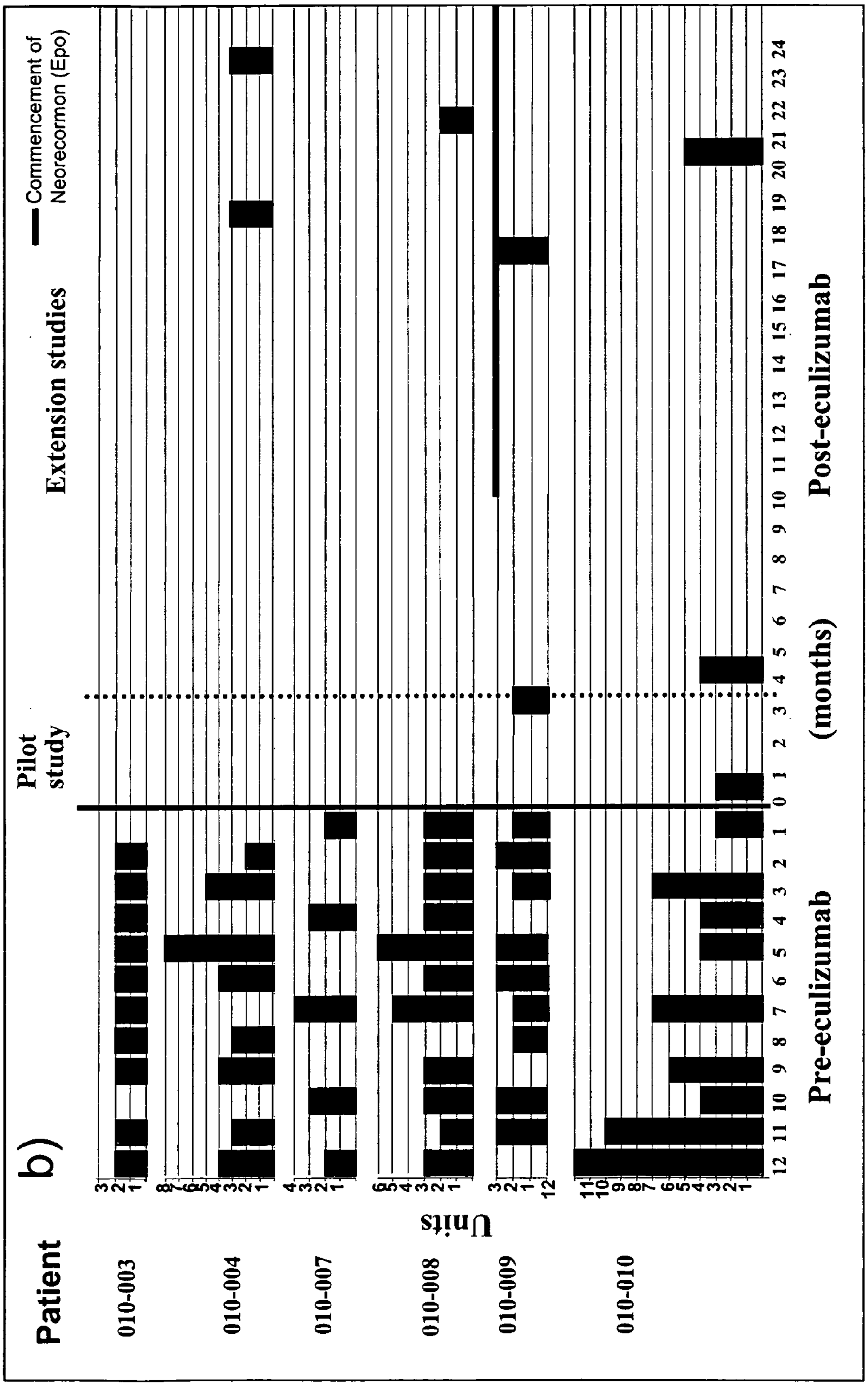

## Figure 7 Management of thrombocytopenic patient with erythropoietin

### Patient 010-001 (hypoplastic; platelets 80 to 100 x $10^9$/l)

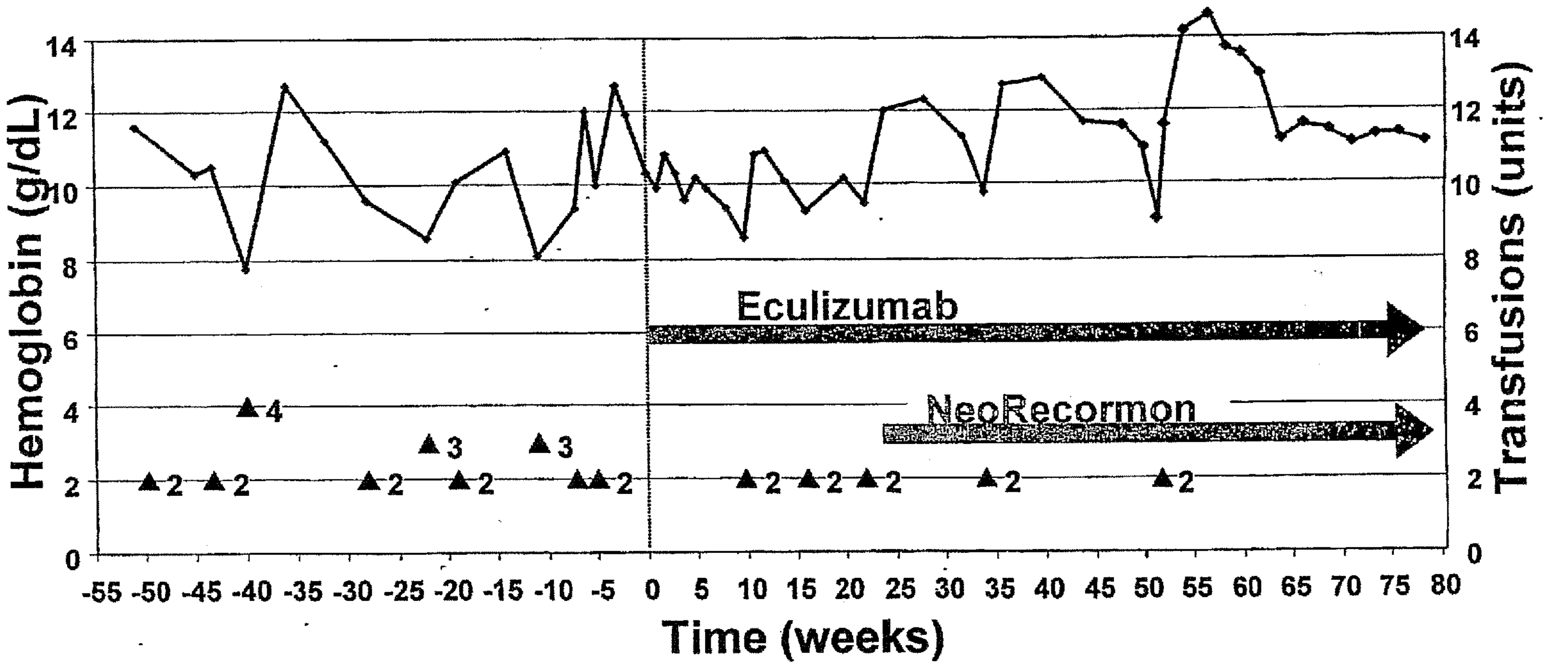


Transfusions reduced with eculizumab and transfusion independence with combined eculizumab and erythropoietin (NeoRecormon 18,000U 3x/week)

Figure 8.
Eculizumab Pharmacodynamics Pre- and Post-dose

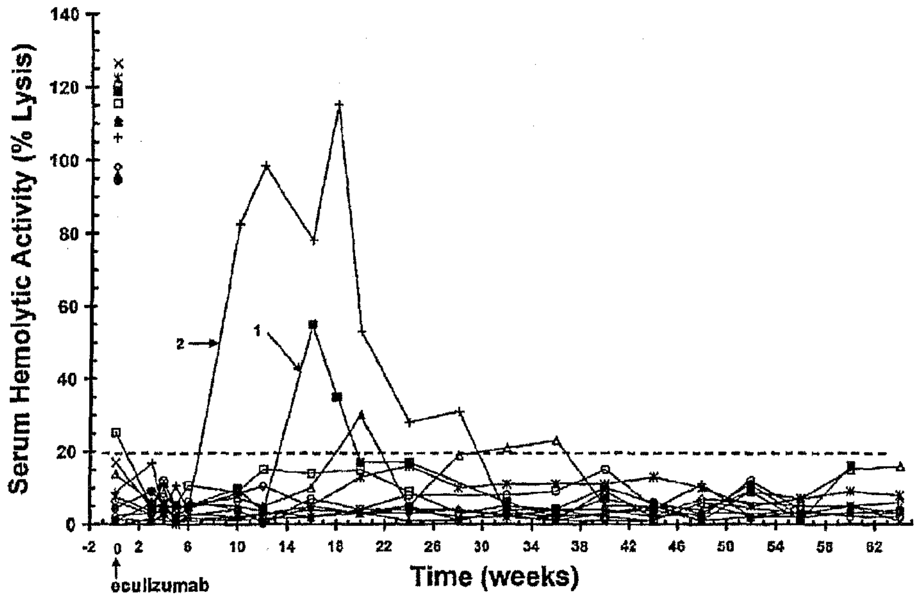


Figure 8 shows serum hemolytic activity (PD) during the 64 week treatment period as determined by the ability of serum to lyse antibody-presensitized chicken erythrocytes. The percentage of hemolytic activity at which complement is considered to be effectively inhibited (≤ 20 %) is indicated by dashed line. Two patients that demonstrated trough serum hemolytic activity values less than 20% are identified (patients 1 and 2).

Figure 9.
Eculizumab Pharmacodynamics Pre- and Post-dose

| Domain (a) | Mean Base-Line Score (b) | 64 Week Change from Base-line (c) | p-value (d) |
|---|---|---|---|
| Global Health Status | 56.1 | 13.8 | 0.009 |
| Physical Functioning | 70.9 | 14.3 | <0.001 |
| Emotional Functioning | 70.5 | 12.5 | <0.001 |
| Role Functioning | 66.7 | 14.5 | 0.003 |
| Cognitive Functioning | 77.3 | 10.3 | 0.001 |
| Fatigue | 47.5 | -17.8 | <0.001 |
| Dyspnea | 39.4 | -16.6 | 0.002 |
| Insomnia | 30.3 | -8.2 | 0.031 |
| Pain | 21.2 | -8.2 | 0.023 |
| Constipation | 3.0 | 4.1 | <0.001 |

a) the quality of life was assessed using the European Organization for Research and Treatment of Cancer QLQ-C30 instrument
b) numbers represent mean values of linearly transformed scores
c) values represent least-square means; positive change indicates improvement for Global Health Status and Functional Scales while a negative change indicates improvement for Symptom Scales
d) values are from a mixed analysis-of-covariance model with visit as a fixed effect, patient as a random effect, and baseline as a covariate

## Figure 10.
## Symptoms Pre- and 2 Years Post-Eculizumab

| Patient | Pre-Eculizumab | | | Post-Eculizumab | | |
|---|---|---|---|---|---|---|
| | Abdominal Pain | Dysphagia | Erectile Dysfunction | Abdominal Pain | Dysphagia | Erectile Dysfunction |
| 010-001 | Every 4 – 8 weeks | Every 4 – 8 weeks | Every 4 – 8 weeks | None | None | None |
| 010-002 | None | None | -- | None | None | -- |
| 010-003 | Every week | Every week | Every week | None | None | None |
| 010-004 | Every 4 – 6 weeks | None | -- | None | None | -- |
| 010-006 | None | None | None | None | None | None |
| 010-007 | None | At least every 4 weeks | -- | None | None | -- |
| 010-008 | None | Every 10 weeks | Every 10 weeks | None | None | Intermittent |
| 010-009 | None | None | None | None | None | None |
| 010-010 | Every 4 weeks | Every 4 weeks | None | None | 2 episodes in 2 years* | None |
| 011-001 | None | None | -- | None | None | -- |
| 011-003 | None | None | -- | None | None | -- |

*This patient experienced a transient breakthrough in complement blockade with a return of hemolysis and symptoms. Increasing the dosing frequency to 12 days re-established complete complement blockade and prevented further symptoms.

Europäisches Patentamt
European Patent Office
Office européen des brevets

# EUROPEAN SEARCH REPORT

Application Number
EP 12 16 9513

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br>Y | ROSSE WENDELL F ET AL: "Immune-mediated hemolytic anemia.", HEMATOLOGY / THE EDUCATION PROGRAM OF THE AMERICAN SOCIETY OF HEMATOLOGY. AMERICAN SOCIETY OF HEMATOLOGY. EDUCATION PROGRAM 2004, January 2004 (2004-01), pages 48-62, XP002497825, ISSN: 1520-4391<br>* the whole document *<br>----- | 1-3,6-9<br><br><br><br><br><br><br><br><br>5 | INV.<br>A61K39/395<br>C07K16/18<br>A61K45/06 |
| A | JANE SALODOF MACNEIL: "Eculizumab could be paroxysmal nocturnal hemoglobinuria breakthrough", 19000101 , 13 December 2002 (2002-12-13), pages 1-2, XP009106338, Medscape Medical News Retrieved from the Internet: URL:http://bcbsma.medscape.com/viewarticle/446336 [retrieved on 2008-09-24]<br>* the whole document *<br>----- | 1-9 | TECHNICAL FIELDS SEARCHED (IPC)<br>A61K<br>C07K |
| A | "Alexion receives FDA, European approval for orphan drug status of new treatment for paroxysmal nocturnal hemoflobinuria", TRANSPLANT NEWS , 15 January 2004 (2004-01-15), XP002497241, Retrieved from the Internet: URL:http://findarticles.com/p/articles/mi_m0YUG/is_1_14/ai_n17208612 [retrieved on 2008-09-25]<br>* the whole document *<br>-----<br>-/-- | 1-9 | |

The present search report has been drawn up for all claims

1

| Place of search | Date of completion of the search | Examiner |
|---|---|---|
| Munich | 8 October 2012 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 9513

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HALL CLAIRE ET AL: "Primary prophylaxis with warfarin prevents thrombosis in paroxysmal nocturnal hemoglobinuria (PNH)", BLOOD, AMERICAN SOCIETY OF HEMATOLOGY, US, vol. 102, no. 10, 15 November 2003 (2003-11-15), pages 3587-3591, XP002482083, ISSN: 0006-4971 *The whole document, in particular the abstract* ----- | 1-9 | |
| A | THOMAS T C ET AL: "Inhibition of complement activity by humanized anti-C5 antibody and single-chain Fv", MOLECULAR IMMUNOLOGY, ELMSFORD, NY, US, vol. 33, no. 17-18, 1 January 1996 (1996-01-01), pages 1389-1401, XP002262113, ISSN: 0161-5890 * the whole document * ----- | 1-9 | TECHNICAL FIELDS SEARCHED (IPC) |
| X,P | HILLMEN PETER ET AL: "Effect of eculizumab on hemolysis and transfusion requirements in patients with paroxysmal nocturnal hemoglobinuria.", THE NEW ENGLAND JOURNAL OF MEDICINE 5 FEB 2004, vol. 350, no. 6, 5 February 2004 (2004-02-05), pages 552-559, XP009057948, ISSN: 1533-4406 | 1-4,6-9 | |
| Y,P | * the whole document * ----- | 5 | |
| | -/-- | | |

The present search report has been drawn up for all claims

1

| Place of search | Date of completion of the search | Examiner |
|---|---|---|
| Munich | 8 October 2012 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 9513

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | FITCH JANE C K ET AL: "Pharmacology and biological efficacy of a recombinant, humanized, single-chain antibody C5 complement inhibitor in patients undergoing coronary artery bypass graft surgery with cardiopulmonary bypass", CIRCULATION, LIPPINCOTT WILLIAMS & WILKINS, US, vol. 100, no. 25, 21 December 1999 (1999-12-21), pages 2499-2506, XP002209328, ISSN: 0009-7322 * the whole document * ----- | 1-9 | |
| T | HILL A ET AL: "Improvemnet in the symptons of smooth muscle dystonia during eculi therapy in paroxysmal nocturnal hemoglobinuria", PUBMED, 1 December 2005 (2005-12-01), - 31 December 2005 (2005-12-31), XP003000273, * the whole document * ----- | | TECHNICAL FIELDS SEARCHED (IPC) |
| A | HILLMEN P ET AL: "Eculizumab, a C5 complement blocking antibody, is the first therapy to reduce transfusion requirements in patients with paroxysmal nocturnal haemoglobinuria (PNH)", BRITISH JOURNAL OF HAEMATOLOGY, WILEY-BLACKWELL PUBLISHING LTD, GB, vol. 121, no. Suppl.1, 1 May 2003 (2003-05-01), page 87, XP009144758, ISSN: 0007-1048 * the whole document * ----- -/-- | 1-9 | |

The present search report has been drawn up for all claims

1

| Place of search | Date of completion of the search | Examiner |
|---|---|---|
| Munich | 8 October 2012 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 16 9513

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KAPLAN M: "ECULIZUMAB", CURRENT OPINION IN INVESTIGATIONAL DRUGS, PHARMAPRESS, US, vol. 3, no. 7, 1 July 2002 (2002-07-01), pages 1017-1023, XP009045356, ISSN: 1472-4472 * the whole document * ----- | 5 | |
| | | | TECHNICAL FIELDS SEARCHED (IPC) |

The present search report has been drawn up for all claims

1

| Place of search | Date of completion of the search | Examiner |
|---|---|---|
| Munich | 8 October 2012 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 77155204 A
- US 6355245 B
- US 4686100 A
- EP 0411306 A
- US 5135916 A, Sims


### Non-patent literature cited in the description


- **WURZNER et al.** *Complement Inflamm.,* 1991, vol. 8, 328-340
- **HAVILAND et al.** *J. Immunol.,* 1991, vol. 146, 362-368
- **MINTA ; MAN.** *J. Immunol.,* 1977, vol. 119, 1597-1602
- **WETSEL ; KOLB.** *J. Immunol.,* 1982, vol. 128, 2209-2216
- **YAMMAMOTO ; GEWURZ.** *J. Immunol.,* 1978, vol. 120, 2008
- **DAMERAU et al.** *Molec. Immunol.,* 1989, vol. 26, 1133-1142
- **MOONGKARNDI et al.** *Immunobiol.,* 1982, vol. 162, 397
- **MOONGKARNDI et al.** *Immunobiol.,* 1983, vol. 165, 323
- **MOLLNES et al.** *Scand. J. Immunol.,* 1988, vol. 28, 307-312
- **AMES et al.** *J. Immunol.,* 1994, vol. 152, 4572-4581
- **GICLAS et al.** *J. Immunol. Meth.,* 1987, vol. 105, 201-209
- **THOMAS et al.** inhibition of Complement Activity by Humanized Anti-C5 Antibody and Single Chain Fv. *Molecular Immunology,* 1996, vol. 33 (17-18), 1389-1401
- **JOHNE et al.** *J. Immunol. Meth.,* 1993, vol. 160, 191-198
- **RICHARDS et al.** *Clin. Appl. Immunol. Rev.,* 2001, vol. 1, 315-330
- **MOYO et al.** *British J. Haematol.,* 2004, vol. 26, 133-138